(19) Europäisches Patentamt
European Patent Office
Office européen des brevets

(11) EP 1 645 580 A2

(12) # EUROPÄISCHE PATENTANMELDUNG

(43) Veröffentlichungstag:
**12.04.2006 Patentblatt 2006/15**

(51) Int Cl.:
*C08G 18/32* (2006.01) *C08G 18/50* (2006.01)
*C08G 18/61* (2006.01) *C08G 18/08* (2006.01)

(21) Anmeldenummer: **05027700.3**

(22) Anmeldetag: **09.03.2000**

(84) Benannte Vertragsstaaten:
**AT BE CH CY DE DK ES FI FR GB GR IE IT LI LU MC NL PT SE**
Benannte Erstreckungsstaaten:
**RO SI**

(30) Priorität: **12.03.1999 DE 19910996**

(62) Dokumentnummer(n) der früheren Anmeldung(en) nach Art. 76 EPÜ:
**00104984.0 / 1 035 144**

(71) Anmelder: **BASF Aktiengesellschaft**
**67056 Ludwigshafen (DE)**

(72) Erfinder:
- **Kim, Son Nguyen, Dr.**
  **69502 Hemsbach (DE)**
- **Sanner, Axel, Dr.**
  **67227 Frankenthal (DE)**
- **Hössel, Peter, Dr.**
  **67105 Schifferstadt (DE)**

Bemerkungen:
Diese Anmeldung ist am 17 -12 - 2005 als Teilanmeldung zu der unter INID-Kode 62 erwähnten Anmeldung eingereicht worden.

(54) **Polyharnstoffe**

(57) Die vorliegende Erfindung betrifft Polyharnstoffe, ein kosmetisches oder pharmazeutisches Mittel, das wenigstens einen solchen Polyharnstoff enthält, sowie die Verwendung der Polyharnstoffe.

EP 1 645 580 A2

## Beschreibung

Die vorliegende Erfindung betrifft Polyharnstoffe, die Verwendung dieser Polyharnstoffe sowie Mittel, die diese Polyharnstoffe enthalten.

In der Kosmetik werden Polymere mit filmbildenden Eigenschaften zur Festigung, Strukturverbesserung und Formgebung der Haare verwendet. Diese Haarbehandlungsmittel enthalten im Allgemeinen eine Lösung des Filmbildners in einem Alkohol oder einem Gemisch aus Alkohol und Wasser.

Haarbehandlungsmittel, insbesondere Haarfestigungsmittel werden im Allgemeinen in Form von wäßrig-alkoholischen Lösungen auf die Haare aufgesprüht. Nach dem Verdampfen des Lösungsmittels werden die Haare an den gegenseitigen Berührungspunkten vom zurückbleibenden Polymer in der gewünschten Form gehalten. Die Polymere sollen einerseits so hydrophil sein, daß sie aus dem Haar ausgewaschen werden können, andererseits aber sollen sie hydrophob sein, damit die mit den Polymeren behandelten Haare auch bei hoher Luftfeuchtigkeit ihre Form behalten und nicht miteinander verkleben. Um eine möglichst effiziente Haarfestigerwirkung zu erzielen, ist es außerdem wünschenswert, Polymere einzusetzen, welche ein relativ hohes Molekulargewicht und eine relativ hohe Glastemperatur (mindestens 10°C) besitzen.

Ein weiterer aktueller Anspruch an Haarbehandlungsmittel ist es, dem Haar Flexibilität, ein natürliches Aussehen und Glanz zu verleihen, z.B. auch dann, wenn es sich um von Natur aus besonders kräftiges und/oder dunkles Haar handelt.

Bei der Formulierung von Haarfestigern ist außerdem zu berücksichtigen, daß aufgrund der Umweltbestimmungen zur Kontrolle der Emission flüchtiger organischer Verbindungen (VOC = volatile organic compounds) in die Atmosphäre eine Verringerung des Alkohol- und Treibmittelgehalts erforderlich ist.

Es ist bekannt, wasserlösliche oder dispergierbare Polyurethane in der Kosmetik einzusetzen. So eignen sie sich z.B. aufgrund ihrer filmbildenden Eigenschaften und einer im Allgemeinen niedrigen Viskosität in Wasser/Ethanol für den Einsatz in der Haarkosmetik, wie z.B. zur Formulierung von lösungsmittelarmen Haarsprays.

Die DE-A-42 25 045 und die WO 94/03515 beschreiben die Verwendung von wasserlöslichen oder in Wasser dispergierbaren, anionischen Polyurethanen als Haarfestiger.

Die in diesen Polyurethanen enthaltenen Säuregruppen können durch Neutralisation mit wenigstens einer Base in die entsprechenden Salze überführt werden. Dazu werden niedermolekulare Amine, wie 2-Amino-2-methylpropanol, Diethylaminopropylamin und Triisopropanolamin eingesetzt.

Die EP-A-619 111 beschreibt die Verwendung von Polyurethanen auf Basis von organischen Diisocyanaten, Diolen und 2,2-Hydroxymethyl-substituierten Carboxylaten in Haarfixierungsmitteln. Zumindest ein Teil der Carbonsäuregruppen wird dabei mit einer organischen oder anorganischen niedermolekularen Base neutralisiert.

Die DE-A-195 41 658 beschreibt wasserlösliche bzw. wasserdispergierbare Pfropfpolymere aus einem Polyurethanpräpolymer mit endständigen Isocyanatgruppen und einem freie Aminogruppen enthaltenden Protein.

Die EP-A-636 361 beschreibt eine kosmetische Zusammensetzung, welche in einem kosmetisch verträglichen Träger mindestens einen Pseudolatex auf Basis eines Polykondensates umfasst, das mindestens eine Polysiloxaneinheit und mindestens eine Polyurethan- und/oder Polyharnstoffeinheit mit anionischen oder kationischen Gruppen umfasst. Als Neutralisierungsmittel werden dabei Mineralbasen, niedermolekulare Amine und Aminoalkohole, Mineralsäuren und niedermolekulare Carbonsäuren eingesetzt. Die WO 97/25021 hat einen vergleichbaren Offenbarungsgehalt. Die Auswaschbarkeit dieser Filmbildner ist nicht zufriedenstellend. Zudem besitzen sie aufgrund eines hohen Siloxananteils auch nicht die für ein Haarpolymer erforderliche Festigungswirkung.

Die DE-A-195 41 329 und die WO 97/17052 beschreiben Haarbehandlungsmittel, enthaltend ein in Wasser oder in einem Wasser/Alkohol-Gemisch lösliches oder dispergierbares Haarfestigerpolymer und zusätzlich ein in Wasser lösliches oder dispergierbares siloxanhaltiges Salz. Haarspray-Formulierungen auf Basis dieser siloxanhaltigen Salze, einem nicht siloxanhaltigen Haarfestigerpolymer und einem Siliconöl führen zu sehr glatten Filmen.

Die DE-A-195 41 326 und die WO 97/17386 beschreiben wasserlösliche oder wasserdispergierbare Polyurethane mit endständigen Säuregruppen, ihre Herstellung und ihre Verwendung. Dabei wird ein in Wasser lösliches oder dispergierbares Polyurethanpräpolymer mit endständigen Isocyanatgruppen mit einer Aminosulfonsäure oder Aminocarbonsäure, insbesondere Taurin, Asparaginsäure und Glutaminsäure, umgesetzt.

Die DE-A-197 09 277 betrifft polysiloxanhaltige Haarfestigungsmittel, enthaltend 0,5 bis 15 Gew.-% carboxylgruppenhaltige Polymere, die in neutralisierter Form wasserlöslich oder wasserdispergierbar sind. Als Neutralisierungsmittel werden dabei Alkalicarbonate, Ammoniak sowie Amine und Aminoalkohole mit maximal 3 Kohlenstoffatomen in der längsten Kohlensfoffkette eingesetzt.

Keines der zuvor genannten Dokumente beschreibt polymere Salze aus einem polymeren Kation und einem polymeren Anion, wobei Polyharnstoff oder Polyurethan, das polymere Anion und/oder das polymere Kation ist. Die zuvor beschriebenen Polyurethane führen zu Filmen, die in Bezug auf ihre Flexibilität und somit in Bezug auf die dem Haar verliehene Geschmeidigkeit noch verbesserungswürdig sind.

Es ist bekannt, Copolymere auf der Basis $\alpha,\beta$-ethylenisch ungesättigter Mono- und/oder Dicarbonsäuren in

Haarpflegemitteln einzusetzen.

Die GB-A-1 321 836 beschreibt Haarfestiger auf der Basis von Copolymeren, die eine ungesättigte Dicarbonsäure und ein Vinyl- oder Vinylidenmonomer einpolymerisiert enthalten. Dabei sind 5 bis 20 % der Carboxylgruppen mit primären $C_4$- bis $C_{16}$-Aminen neutralisiert.

Die DE-A-29 17 504 beschreibt ein Aerosol-Haarspray auf der Basis eines Copolymers aus mindestens einer ungesättigten Monocarbonsäure und mindestens einem Vinyl- oder Vinylidenmonomer. Dabei sind mindestens 7 bis 100 % der Carboxylgruppen neutralisiert, davon mindestens die Hälfte mit einem langkettigen primären, sekundären und/oder tertiären Amin mit 8 bis 20 Kohlenstoffatomen in der längsten Kette.

Die WO 89/12438 beschreibt einen Haarfestiger auf Basis eines Haarpolymers mit Carboxylgruppen, die zu mindestens 40 Mol-% mit einem langkettigen Amin, das ausgewählt ist unter Amidoaminen, N-ethoxylierten Aminen und Etheraminen, neutralisiert sind.

Die zuvor genannten Polyacrylate mit Carbonsäuregruppen, die mit Fettaminen oder ethoxylierten Fettaminen neutralisiert sind, führen zu weichen, klebrigen Filmen mit einer drastisch reduzierten Festigungswirkung. Diese Polymere eignen sich daher nur sehr eingeschränkt für einen Einsatz als Haarfestiger.

Die JP-A-7127480 beschreibt ein Haarbehandlungsmittel auf Basis einer Aminsalz-Lösung eines Copolymers, das eine ungesättigte Carbonsäure einpolymerisiert enthält.

Die JP-A-03206023 beschreibt ein Polymerharz für Haarbehandlungsmittel, welches a) 6 bis 35 Gew.-% Acrylsäure, Methacrylsäure, Itaconsäure oder eine Mischung davon, b) 15 bis 50 Gew.-% wenigstens eines $C_{10}$- bis $C_{18}$-Alkyl (meth)- acrylats, c) 15 bis 50 Gew.-% wenigstens eines $C_4$- bis $C_8$-Alkyl(meth)- acrylats und d) 0 bis 25 Gew.-% wenigstens eines weiteren hydrophoben Vinylmonomers einpolymerisiert enthält. Die erhaltenen Copolymere werden mit einer Base, ausgewählt unter Ammoniak, Morpholin, Isopropanolamin und Aminoethylpropandiol, neutralisiert.

Die JP-A-03206024 beschreibt ein der JP-A-03206023 vergleichbares Haarfestigerpolymer, das zusätzlich 5 bis 50 Gew.-% eines
N-Alkyl-substituierten Acrylamids einpolymerisiert enthält. Die in diesen beiden Dokumenten beschriebenen Haarfestigerpolymere weisen einen hohen Anteil an hydrophoben Monomeren auf. Ihre Auswaschbarkeit ist daher verbesserungswürdig.

Die DE-A-39 01 325 und die DE-A-42 14 305 beschreiben Haarfestigungsmittel, die als Filmbildner ein Copolymerisat auf Basis von tert.-Butyl(meth)acrylat und (Meth)acrylsäure enthalten, wobei die Carboxylgruppen der Copolymerisate teilweise oder vollständig durch Amine neutralisiert sind. Dabei sind die Amine ausgewählt unter Mono-, Di- oder Trialkanolaminen, Alkandiolaminen oder primären, sekundären oder tertiären Alkylaminen. Filme auf Basis dieser Polyacrylate sind im Allgemeinen hart und zeigen keine Flexibilität und insbesondere in Bezug auf ihre Festigungswirkung verbesserungswürdig.

Die DE-A-197 09 277 beschreibt polysiloxanhaltige Haarfestigungsmittel auf Basis von Carboxylgruppen-haltigen Polymeren, Polysiloxanen mit primären, sekundären oder tertiären Aminogruppen sowie niedermolekularen Neutralisierungsmitteln. Der Einsatz von nicht weiter funktionalisierten Siloxandiaminen in Haarfestigungsmitteln führt zu vernetzten Produkten, die schlecht auswaschbar sind. Aufgrund der schlechten Haftung ist die Festigungswirkung dieser Formulierungen verbesserungswürdig.

US 4761273 betrifft Gemische aus anionischem und kationischem Polymer, wobei das anionische Polymer ein Polyacrylat oder ein Derivat davon ist und das kationische Polymer ein Polyamin oder ein Derivat davon ist. Diese Polymersalze sind weich und klebriger als die erfindungsgemäßen.

Der vorliegenden Erfindung liegt die Aufgabe zugrunde, Polymere zur Verfügung zu stellen. Diese sollen sich als kosmetisches Mittel, beziehungsweise für einen Einsatz in kosmetischen Mitteln, insbesondere Haarbehandlungsmittel(n), eignen. Vorzugsweise sollen sie Filme mit guten flexiblen Eigenschaften bilden und dabei klebfrei sein, so daß auf ihnen basierende Haarbehandlungsmittel dem Haar Elastizität verleihen. Die erfindungsgemäßen Polymere sollenverleihen dem Haar besondere Eigenschaften verleihen: natürlichen Halt, gute Kämmbarkeit, guter Griff und Geschmeidigkeit. Dies gilt besonders für Polymere für Haarspray-Formulierungen. Für gewisse Haartypen in USA und Asien werden Polymere mit erhöhter Flexibilität gewünscht.

Überraschenderweise wurde nun gefunden, daß diese Aufgabe durch bestimmte Polyharnstoffe sowie wasserlösliche oder wasserdispergierbare polymere Salze gelöst wird.

Gegenstand der vorliegenden Erfindung ist daher ein Polyharnstoff, aufgebaut aus:

a) einem Diamin, das eine Gruppe $-(-CH_2CH_2O-)_n-(C_3H_6-O-)_m$ enthält, wobei die Reihenfolge der Alkylenoxideinheiten beliebig ist und m und n unabhängig voneinander für eine ganze Zahl zwischen 0 und 50 stehen, und m+n zwischen 5 und 60 liegen und

b) mindestens einem aminohaltigen oder hydroxyhaltigen Polysiloxan und

c) mindestens einem Diisocyanat, und

d) optional einem Di-, Tri-, Tetraamin oder Polyamin, das mindestens eine ionogene Gruppe enthält, und

e) optional einem oder mehreren Diaminen mit einem Molekulargewicht von 60 bis 6000 g/mol,

wobei der Polyharnstoff mindestens eine ionogene oder ionische Gruppe enthält.

Bevorzugt sind solche Polyharnstoffe, die mindestens eine freie Amingruppierung enthalten.

Des weiteren betrifft die Erfindung ein Verfahren zur Herstellung der oben genannten Polyharnstoffe, wobei die Polymerisation in Alkohol oder Wasser/Alkohol-Lösung bei einer Temperatur kleiner gleich 50°C durchgeführt wird, bei hydroxyhaltigen Polysiloxanen werden zuerst die Komponenten b) und c) miteinander umgesetzt.

Die Erfindung betrifft außerdem die Verwendung von neutralisiertem Polyharnstoff als filmbildendes Hilfsmittel als Additiv

füor kosmetische Präparate. Des weiteren betrifft die Erfindung die Verwendung von Polyharnstoff mit ionischen oder ionogenen Gruppen als Neutralisationsmittel für Polymere, die ionische oder ionogene Gruppen enthalten.

Gegenstand der Erfindung sind wasserlösliche oder wasserdispergierbare Salze enthaltend

A) ein Grundpolymer, das eine ionische oder ionogene Gruppe enthält und
B) ein Neutralisationspoiymer, das die ionische oder ionogene Gruppe des Grundpolymers A zum Teil neutralisiert, wobei die ionogenen oder ionischen Gruppen von A um einen Faktor 2 bis 30 häufiger sind als die ionogenen Gruppen des Neutralisationspolymers B.

Sowohl das Grundpolymer als auch das Neutralisationspolymer können durch Zugabe von Neutralisationsmitteln (teil)neutralisiert werden. Dabei wird in der Regel zunächst das Grundpolymer durch Zugabe eines Neutralisationsmittels (teil)neutralisiert und dann erfolgt die Zugabe des Neutralisationspolymers. Ebenso ist es möglich das Neutralisationspolymer zu dem Grundpolymer zu zugeben und anschließend mit Neutralisationsmitteln zu neutralisieren. Besonders bevorzugt ist die (Teil)neutralisation des Grundpolymers und die anschließende Zugabe des Neutralisationspolymers. Als Neutralisationsmittel wird vorzugsweise ein Gemisch unterschiedlicher Neutralisationsmittel eingesetzt.

In einer bevorzugten Ausführungsform ist das Grundpolymer A und/oder das Neutralisationspolymer B ein Polyharnstoff oder ein Polyurethan. In einer besonders bevorzugten Ausführungsform ist das Grundpolymer A und/oder das Neutralisationspolymer B ein Polyharnstoff nach Anspruch 1.

Als Neutralisationsmittel können eingesetzt werden:
Bei überschüssigen anionogenen oder anionischen Gruppen wird mit einem Amin oder Amingemisch (z.B. Amino-2-methyl-propanol AMP, Ethylendiamin etc.) und/oder mit einer Base (NaOH, KOH etc.) neutralisiert.

Vorzugsweise werden zur Neutralisation der Polymere mit anionogenen Gruppen Gemische eingesetzt, die wenigstens ein Amin, bevorzugt ein hydroxyhaltiges Amin, wie Amino-2-methylpropanol, Mono-, Di- und Triethanolamin oder ein Diamin wie Ethylendiamin, und ein Alkalimetallhydroxid, bevorzugt Kaliumhydroxid, enthalten. Vorzugsweise werden zur Neutralisation der Polyurethane mit anionogenen Gruppen weiterhin Gemische eingesetzt, die wenigstens ein Amin und einen Aminoalkohol, bevorzugt 2-Amino-2-methyl-1-propanol, enthalten.

Bei überschüssigen kationogenen oder kationischen Gruppen wird mit einer Säure oder Säuremischung (z.B. Milchsäure, Phosphorsäure etc.) neutralisiert.

Die Amine, Säuren oder/und Basen sind bevorzugt kosmetisch verträglich.

Eine Aminzahl kleiner als die Säurezahl ist besonders bevorzugt.

Kosmetisch verträgliche Säuren und Polysäuren sind dem Fachmann bekannt und z.B. in WO 97/17052 aufgeführt und hiermit inkooperiert. Kosmetisch verträgliche Amine sind dem Fachmann ebenfalls bekannt und auch in der vorliegenden Anmeldung aufgeführt.

Das Grundpolymer A kann ein Polyurethan, Polyharnstoff, Poly-(urethan/harnstoff) aufgebaut aus:

f) mindestens einer Verbindung, die zwei (oder mehrere) aktive Wasserstoffatome pro Molekül,

g) mindestens einer Verbindung, die zwei aktive Wasserstoffatome pro Molekül mit Molekulargewicht von 56 bis 300 g/mol,

h) mindestens eine Phosphat-, Phosphonat-,Carboxylat-, Sulfat und/oder tert.-Amin-haltigen Verbindung oder deren Verbindungen, die die freien Säuren enthalten, die zwei aktive Wasserstoffatome pro Molekül enthält und

i) mindestens einem Diisocyanat, sein.

Oder das Grundpolymer A ist ein Polyacrylat, aufgebaut aus:

mindestens einem $C_1$-$C_4$-Alkylacrylsäureester oder $C_1$-$C_4$-Alkylenmmethacrylsäureester oder Acrylamid oder Methacrylamid oder, $C_1$-$C_4$-Alkylacrylamid oder $C_1$-$C_4$-Alkylmethacrylamid,

mindestens einem COOH-haltigen Monomer.

Bevorzugt sind Polyacrylate enthaltend 65 - 90 Gew.-% Ester oder Amid und 10-35 Gew.-% COOH-haltigen Monomer.
Die bevorzugten Ester sind $C_4$-Alkylacrylsäureester bzw. $C_4$-Alkylmethacrylsäureester.
Die bevorzugten Amide sind $C_4$-Alkylacrylsäureamide bzw. $C_4$-Alkylmethacrylsäureamide.
Als Neutralisationspolymer wird bevorzugt ein filmbildendes Polymer eingesetzt, welches mit Wasser dispergierbar ist.
Die Erfindung betrifft außerdem Polyurethan, Poly(urethan-urea) oder Polyharnstoff als Neutralisationspolymer B, aufgebaut aus:

j) mindestens einer Verbindung oder einem Gemisch aus Verbindungen mit wenigstens zwei aktiven Wasserstoffatomen pro Molekül und mit einem Molekulargewicht von 56 bis 6000 g/mol, wobei mindestens eine Verbindung eine der folgenden Gruppen enthält:

- $(-CH_2-CH_2O-)m-(C_3H_6O-)n-$, wobei die Reihenfolge der Alkylenoxydeinheiten beliebig ist und m sowie n unabhängig voneinander für eine ganze Zahl von 0 bis 50 stehen, und die Summe aus m und n in dem Bereich von 5 bis 60 liegt,

- COO-, $-SO_3$- oder

- $N^+$-Gruppierung (quatemierte Amine),

k) eventuell einer Verbindung mit wenigstens zwei aktiven Wasserstoffatomen pro Molekül und mindestens eine ionogene Gruppe,

l) mindestens einem aminohaltigen Poly(dimethylsiloxan) und

m) mindestens einem Diisocyanat,

wobei die Säurezahl oder Aminzahl nicht größer als 60 ist.
In diesem Neutralisationspolymer liegen die Komponenten bevorzugt in folgenden Mengen vor:

j) 10 bis 90 Gew.-%, bevorzugt 15 bis 80 Gew.-%,
k) 0 bis 20 Gew.-%, bevorzugt 0 bis 10 Gew.-%,
l) 0,1 bis 30 Gew.-%, bevorzugt 0,3 bis 20 Gew.-%,
m) 5 bis 30 Gew.-%,

bezogen auf j+k+l+m.
In einer bevorzugten Ausführungsform der Erfindung ist das Neutralisationspolymer aufgebaut aus mindestens einem Vinyllactam und/oder mindestens einem Vinylamid und mindestens einem Amin-haltigen Monomer, wobei die Aminzahl zwischen 1 und 60 liegt.
Insbesondere kann ein solches Neutralisationspolymer aufgebaut sein aus:

a) 25 bis 80 Gew.-% mindestens eines Vinyllactams und/oder Vinylamids, bevorzugt Vinylpyrrolidon und/oder Vinylcaprolactam,

b) 1 bis 20-Gew.-% mindestens eines Amin-haltigen Monomers,

c) 0 bis 40 Gew.-% eines $C_1$-$C_4$-Alkylacrylsäureesters oder $C_1$-$C_4$-Alkylmethacrylsäureesters oder Acrylamid oder Methacrylamid oder $C_1$-$C_4$-Alkylacrylamid oder $C_1$-$C_4$-Alkylmethacrylamid, bevorzugt ein entsprechender $C_4$-Alkyl-Ester und $C_4$-Alkylamide,

wobei die freie Aminzahl zwischen 1 und 60 liegt.
In einer weiteren bevorzugten Ausführungsform ist das Neutralisationspolymer aufgebaut aus mindestens

einem $C_1$-$C_{18}$-Alkylmethacrylsäureester und/oder $C_1$-$C_{18}$-Alkylmethacrylamid und mindestens einem Amin-haltigen und/ oder COOH-haltigen Monomer.

Die Erfindung betrifft auch Verfahren zur Herstellung von oben genannten polymeren Salzen, wobei das Grundpolymer in einem geeigneten Lösungsmittel (Wasser oder Alkohol/Wasser) mit einem einwertigen Neutralisationsmittel teilneutralisiert wird und anschließend das Neutralisationspolymer zugegeben wird.

Bevorzugt ist ein Verfahren zur Herstellung eines polymeren Salzes, bei dem ein Vinyllactam tragendes Polymer als Neutralisationspolymer eingesetzt wird, wobei ein anionisches oder anionogenes Grundpolymer (bevorzugt Carboxylatgruppen tragend) vorzugsweise bei einer Temperatur von etwa 80°C etwa 1 h in Wasser oder Alkohol/Wasser neutralisiert wird und anschließend mit niedermolekularem Amin neutralisiert wird. Die organischen Lösungsmittel können nach dem Zusatz von Wasser z.B. durch Destillation unter vermindertem Druck entfernt werden. Die wäßrige Lösung oder Dispersion des polymeren Salzes kann zur Gewinnung des polymeren Salzes verwendet werden (z.B. durch Sprühtrocknung).

Die erfindungsgemäßen polymeren Salze sind zumindest teilweise vernetzt. Die Vernetzung erfolgt dabei über Ionenbindung zwischen anionischen Gruppen von mindestens zwei verschiedenen Polymerketten eines Polymers (Grund- oder Neutralisationspolymer) und wenigstens zwei kationischen Gruppen des anderen Polymers (Neutralisations- oder Grundpolymer).

Die Amine weisen immer wenigstens zwei zur Ausbildung von kationischen Gruppen befähigte primäre, sekundäre und/oder tertiäre Aminogruppen auf.

Bei der Komponente f) des Grundpolymers auf Basis von Polyurethanen handelt es sich bevorzugt um ein Polymerisat mit einem zahlenmittleren Molekulargewicht im Bereich von etwa 300 bis 5000, bevorzugt etwa 400 bis 4000, insbesondere 500 bis 3 000 g/mol. Brauchbare Polymerisate f) sind z.B, Polyesterdiole, Polyetherole, Polyamiddiamine, Polysiloxanpolyol/-polyamine und Mischungen davon. Polyetherole sind vorzugsweise Polyalkylenglykole, z.B. Polyethylenglykole, Polypropylenglykole, Polytetrahydrofurane etc., Copolymerisate aus Ethylenoxid und Propylenoxid oder Blockcopolymerisate aus Ethylenoxid, Propylenoxid und Butylenoxid, die die Alkylenoxideinheiten statistisch verteilt oder in Form von Blöcken einpolymerisiert enthalten. Geeignet sind auch $\alpha,\omega$-Diaminopolyether, die durch Aminierung von Polyalkylenoxiden mit Ammoniak herstellbar sind. Vorzugsweise werden als Komponente f) Polyesterdiole und Mischungen, die diese enthalten, eingesetzt.

Geeignete Polytetrahydrofurane können durch kationische Polymerisation von Tetrahydrofuran in Gegenwart von sauren Katalysatoren, wie z.B. Schwefelsäure oder Fluoroschwefelsäure, hergestellt werden. Derartige Herstellungsverfahren sind dem Fachmann bekannt.

Bevorzugte Polyesterdiole weisen ein zahlenmittleres Molekulargewicht im Bereich von etwa 400 bis 5000, bevorzugt 500 bis 3000, insbesondere 600 bis 2000, auf.

Als Polyesterdiole kommen alle diejenigen in Betracht, die üblicherweise zur Herstellung von Polyurethanen eingesetzt werden, insbesondere solche auf Basis aromatischer Dicarbonsäuren, wie Terephthalsäure, Isophthalsäure, Phthalsäure, Na- oder K-Sulfoisophthalsäure etc., aliphatischer Dicarbonsäuren, wie Adipinsäure oder Bernsteinsäure etc., und cycloaliphatischer Dicarbonsäuren, wie 1,2-, 1,3- oder 1,4-Cyclohexandicarbonsäure. Als Diole kommen insbesondere aliphatische Diole in Betracht, wie Ethylenglykol, Propylenglykol, 1,6-Hexandiol, Neopentylglykol, Diethylenglykol, Polyethylenglykole, Polypropylenglykole, 1,4-Dimethylolcyclohexan.

Bevorzugt sind Polyesterdiole auf Basis von aromatischen und aliphatischen Dicarbonsäuren und aliphatischen Diolen, insbesondere solche, bei denen die aromatische Dicarbonsäure 10 bis 95 Mol-%, insbesondere 40 bis 90 Mol-% des gesamten Dicarbonsäureanteils (Rest aliphatische Dicarbonsäuren) ausmacht.

Besonders bevorzugte Polyesterdiole sind die Umsetzungsprodukte
Phthalsäure und Diethylenglykol,
Isophthalsäure/Adipinsäure und 1,6-Hexandiol und/oder Neopentylglykol,
Isophthalsäure/Adipinsäure und 1,4-Dimethylolcyclohexan und/oder Neopenthylglykol
Isophthalsäure/Adipinsäure und 1,4-Dimethylolcyclohexan und/oder Dimethylolcyclohexan und/oder Diethylenglykol,
Adipinsäure und 1,6-Hexandiol und/oder Neopentylglykol und/oder 1,4-Dimethylolcyclohexan und/oder Diethylenglykol,
Polyamiddiamin, insbesondere Diamin aus itaconsäure/aliphatische Diamine z B. Itaconsäure/Hexandiamin (1,6).

Bei den Polysiloxanen handelt es sich vorzugsweise um eine Verbindung der Formel I

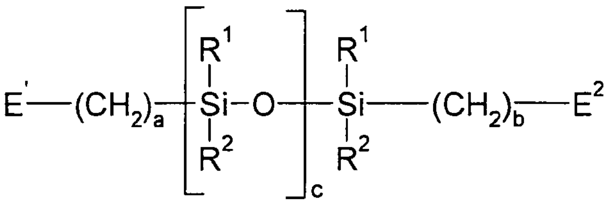

(I)

worin

$R^1$ und $R^2$ unabhängig voneinander für $C_1$- bis $C_4$-Alkyl, Benzyl oder Phenyl stehen,

$E^1$ und $E^2$ unabhängig voneinander für OH oder $NHR^3$ stehen, wobei $R^3$ für Wasserstoff, $C_1$- bis $C_6$-Alkyl oder $C_5$- bis $C_8$-Cycloalkyl steht,

a und b unabhängig voneinander für 2 bis 8 stehen,

c für 3 bis 50 steht,

und Mischungen davon.

Bei den aminohaltigen Polysiloxanen ist zumindest $E^1$ oder $E^2$ gleich $NHR^3$.

Bei den hydroxyhaltigen Polysiloxanen ist zumindest $E^1$ oder $E^2$ gleich -OH.

Geeignete Alkylreste sind z.B. Methyl, Ethyl, n-Propyl, Isopropyl, n-Butyl, t.-Butyl, n-Pentyl, n-Hexyl etc. Geeignete Cycloalkylreste sind z.B. Cyclopentyl, Cyclohexyl, Cycloheptyl, Cyclooctyl etc.

Vorzugsweise stehen $R^1$ und $R^2$ beide für Methyl.

Diese Polysiloxane weisen vorzugsweise ein zahlenmittleres Molekulargewicht im Bereich von etwa 300 bis 5 000, bevorzugt 400 bis 3 000, auf.

Bei den Polysiloxanen handelt es sich weiterhin vorzugsweise um eine Verbindung der Formel II

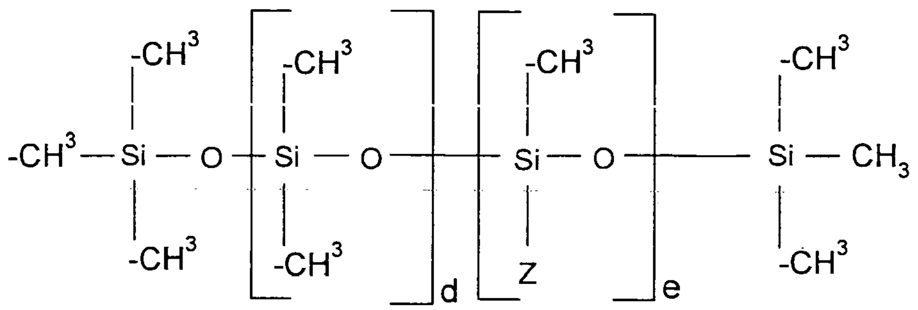

(II)

worin die Reihenfolge der Siloxaneinheiten beliebig ist,

d für einen Wert von 5 bis 200, bevorzugt 10 bis 100, steht,

e für einen Wert von 1 bis 20, bevorzugt 2 bis 10, steht,

Z für einen Rest der Formel $-(CH_2)_f-NH_2$ steht, worin f für eine ganze Zahl von 1 bis 10, bevorzugt 2 bis 6 steht, oder

Z für einen Rest der Formel $-(CH_2)_g-NH-(CH_2)_h-NH_2$ steht, worin g und h unabhängig voneinander für 0 bis 6, bevorzugt 2 bis 3, stehen, oder.

z für $(CH_2)_f-(CH_2-CH_2O)n-(C_3H_6O)_m-H$, worin n + m im Bereich zwischen 5 und 60 liegen und m und n unabhängig voneinander zwischen 0 und 50 liegen.

Dazu zählen z.B. die MAN- und MAR-Marken der Fa. Degussa-Hüls sowie die Finish-Marken der Fa. Wacker, z.B. Finish WT 1270 und Belsil 6031 der Fa. Wacker.

Geeignete Polysiloxane sind auch die in der EP-A-227 816 beschriebenen Polydimethylsiloxane, auf die hiermit Bezug genommen wird.

Geeignete Diamine sind z.B. Ethylendiamin, Propylendiamin, 1,4-Diaminobutan, 1,5-Diaminopentan und 1,6-Diaminohexan.

Als Polyamine sind Verbindungen der folgenden Formel geeignet:

$$HR^3N\text{-}R^5\text{-}(N\text{-}R^7)_p — NHR^4$$
$$|$$
$$R^6$$

worin

p für eine ganze Zahl von 0 bis 4 steht,
$R^3$ und $R^4$ unabhängig voneinander für Wasserstoff, $C_1$- bis $C_{40}$-Alkyl oder $C_6$- bis $C_{40}$-Alkenyl stehen wobei die Alkyl- und Alkenylreste wenigstens eine ionogene und/oder ionische Gruppe tragen können, die ausgewählt ist unter -COOY, $-SO_3Y$ und $-PO_3Y$, wobei Y für H, Li, Na, K oder Ammonium steht, wobei für p =0 wenigstens einer der Reste $R^3$ oder $R^4$ für einen $C_1$- bis $C_{40}$-Alkyl- oder $C_6$- bis $C_{40}$-Alkenylrest steht, der wenigstens eine ionogene und/oder ionische Gruppe trägt,
$R^5$ und $R^7$ für einen $C_2$- bis $C_6$-Alkylenrest stehen, wobei für p > 1 die Reste $R^7$ unabhängig unter $C_2$- bis $C_6$-Alkylenresten ausgewählt sind,
$R^6$ für $C_1$- bis $C_6$-Alkyl, $C_5$- bis $C_8$-Cycloalkyl, Phenyl oder Phenyl-$C_1$-$C_4$-alkyl steht,

wobei für p > 1 die Reste $R^6$ unabhängig unter diesen Bedeutungen ausgewählt sind.

Weist das Polyamin der obigen Formel mehrere Wiederholungseinheiten $(N(R^6)\text{-}R^7)p$ auf, so können diese gleiche oder verschiedene Bedeutungen haben.

Bevorzugt steht p für 1, 2 oder 3, insbesondere für 1 oder 2.

Wenn p für 0 steht, so stehen bevorzugt die Reste $R^3$ und $R^4$ unabhängig für einen $C_1$-bis $C_{40}$-Alkyl- oder $C_6$-bis $C_{40}$-Alkenylrest, der jeweils wenigstens eine ionogene und/oder ionische Gruppe trägt.

Bevorzugt stehen $R^3$ und $R^4$ unabhängig voneinander für Wasserstoff, $C_1$- bis $C_{30}$-Alkyl, bevorzugt $C_1$- bis $C_{12}$-Alkyl, insbesondere $C_1$- bis $C_8$-Alkyl oder einen Rest der $-(CH_2)_{2\text{-}6}—SO_3Y,$ $SO_3Y$, wobei Y für H, Li, Na, K oder Ammonium steht.

Insbesondere stehen $R^3$ und $R^4$ unabhängig voneinander für Wasserstoff, Methyl, Ethyl, n-Propyl, Isopropyl, n-Butyl, sec.-Butyl, tert.-Butyl, n-Pentyl, n-Hexyl. Speziell stehen $R^3$ und $R^4$ beide für Wasserstoff.

Bevorzugt steht $R^5$ für einen $C_2$- bis $C_4$-Alkylenrest.

Bevorzugt steht $R^6$ für Methyl, Ethyl, n-Propyl, Isopropyl, n-Butyl, sec.-Butyl, tert.-Butyl, Cyclohexyl, Phenyl oder Benzyl.

Bevorzugt steht $R^7$ für einen $C_2$- bis $C_4$-Alkylenrest.

Als Polyamin wird vorzugsweise Diethylentriamin, N-Methyldiethylentriamin, N-Ethyldiethylentriamin, N,N,N',N'-Tetramethyldiethylentriamin, N,N,N',N'-Tetraethyldiethylentriamin, Dipropylentriamin, N-Methyldipropylentriamin, N-Ethyldipropylentriamin, N,N'-Bis(3-aminopropyl)-butan-1,4-diamin, Triethylentetramin, Tetraethylenpentamin und Mischungen davon eingesetzt. Besonders bevorzugt handelt es sich bei dem Polyamin um N-Methyldinrooylentriamin.

Bei der Komponente g) des Grundpolymers mit zwei aktiven Wasserstoffatomen handelt es sich bevorzugt um Diole, Diamine, Aminoalkohole, und Mischungen davon. Das Molekulargewicht dieser Verbindungen liegt in einem Bereich von etwa 56 bis 300.

Gewünschtenfalls können bis zu 3 Mol-% der genannten Verbindungen durch Triole oder Triamine ersetzt sein.

Bevorzugt werden hierbei Diole eingesetzt. Brauchbare Diole sind z.B. Ethylenglykol, Propylenglykol, Butylenglykol, Neopentylglykol, Cyclohexandimethylol, Di-, Tri-, Tetra-, Penta- oder Hexaethylenglykol und Mischungen davon. Bevorzugt werden Neopentylglykol und/oder Cyclohexandimethylol eingesetzt.

Geeignete Aminoalkohole sind z.B. 2-Aminoethanol, 2-(N-Methylamino)ethanol, 3-Aminopropanol, 4-Aminobutanol, 1-Ethylaminobutan-2-ol, 2-Amino-2-methyl-1-propanol, 4-Methyl-4-aminopentan-2-ol etc.

Geeignete Diamine sind z.B. Ethylendiamin, Propylendiamin, 1,4-Diaminobutan, 1,5-Diaminopentan und 1,6-Diaminohexan sowie Fettdiamine der Struktur $R\text{-}NH\text{-}(CH_2)_{2\text{-}3}\text{-}NH_2$ mit R = $C_8$-$C_{22}$-Alkyl oder $C_8$-$C_{22}$-Alkenyl-Rest.

Geeignete Verbindungen h) des Grundpolymers weisen zwei aktive Wasserstoffatome und mindestens eine anionogene und/oder anionische Gruppe pro Molekül auf bzw. mindestens eine kationogene und/oder kationische Gruppe pro Molekül auf.

Bevorzugte Verbindungen mit zwei aktiven Wasserstoffatomen und mindestens einer anionogenen und/oder anionischen Gruppe pro Molekül sind z.B. Verbindungen mit Cärboxylat- und/oder Sulfonatgruppen. Als Komponente mit zwei aktiven Wasserstoffatomen sind 2,2-Hydroxymethyl-substituierte Carboxylate, insbesondere Dimethylolpropansäure und Mischungen, die Dimethylolpropansäure enthalten, besonders bevorzugt.

Als Komponente mit zwei aktiven Wasserstoffatomen brauchbar sind auch Verbindungen der Formel

$$H_2N(CH_2)_w\text{-}NH\text{-}(CH_2)_x\text{-}COO\text{-}M^+$$

$$H_2N(CH_2)_w\text{-}NH\text{-}(CH_2)_x\text{-}SO_3\text{-}M^+$$

worin w und x unabhängig voneinander für eine ganze Zahl von 1 bis 8, insbesondere 1 bis 6, stehen und M für Li, Na oder K steht, und Verbindungen der Formel

$$H_2N(CH_2CH_2O)_y(CH_2CH(CH_3)O)_z(CH_2)_w\text{-}NH\text{-}(CH_2)_x\text{-}SO_3\text{-}M^+$$

worin w und x die zuvor angegebenen Bedeutungen besitzen, y und z unabhängig voneinander für eine ganze Zahl von 0 bis 50 stehen, wobei wenigstens eine der beiden Variablen y oder z > 0 ist. Die Reihenfolge der Alkylenoxideinheiten ist dabei beliebig. Die zuletzt genannten Verbindungen weisen vorzugsweise ein zahlenmittleres Molekulargewicht im Bereich von etwa 400 bis 3 000 auf. Eine geeignete Verbindung dieses Typs ist z.B. Poly ESP 520 der Fa. Raschig.

Gewünschtenfalls können die Polyurethane zusätzlich oder anstatt den Verbindungen mit anionogenen und/oder anionischen Gruppen auch Verbindungen eingebaut enthalten, die zwei aktive Wasserstoffatome und mindestens eine kationogene und/oder kationische Gruppe, bevorzugt mindestens eine stickstoffhaltige Gruppe, pro Molekül aufweisen. Bevorzugt handelt es sich bei der stickstoffhaltigen Gruppe um eine tertiäre Aminogruppe oder eine quaternäre Ammoniumgruppe. Bevorzugt sind z.B. Verbindungen der allgemeinen Formeln

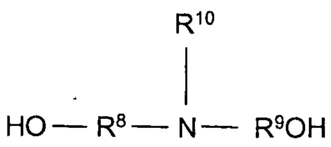
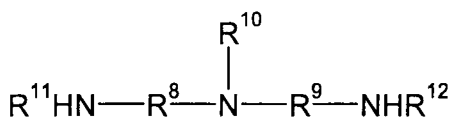

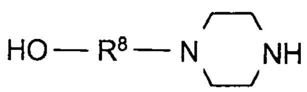
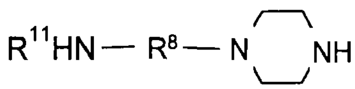

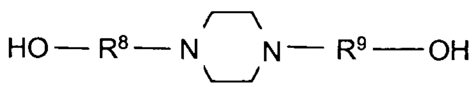
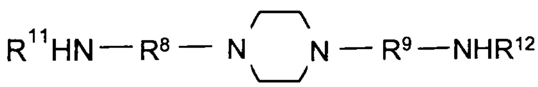

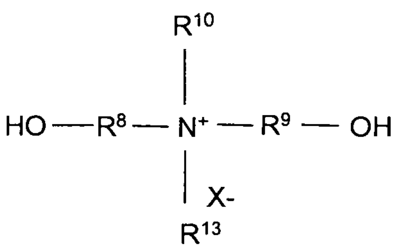
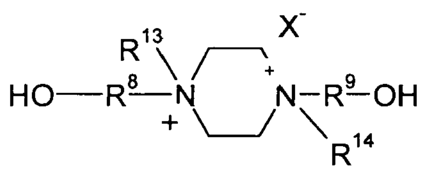

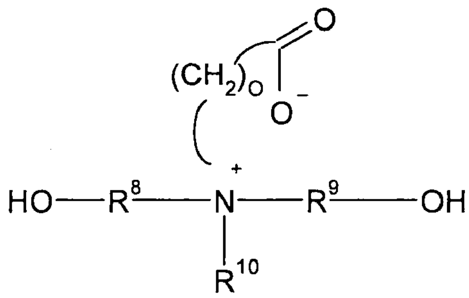

worin

- $R^8$ und $R^9$, die gleich oder verschieden sein können, für $C_2$-$C_8$-Alkylen stehen,
- $R^{10}$, $R^{13}$ und $R^{14}$, die gleich oder verschieden sein können, für $C_1$-$C_6$-Alkyl, Phenyl oder Phenyl-$C_1$-$C_4$-alkyl stehen,
  $R^{11}$ und $R^{12}$, die gleich oder verschieden sein können, für H oder $C_1$-$C_6$-Alkyl stehen,
  o für 1, 2 oder 3 steht,
  $X^{\ominus}$ für Chlorid, Bromid, Jodid, $C_1$-$C_6$-Alkylsulfat oder $SO_4^{2-}/_2$ steht.

Besonders bevorzugt sind N-($C_1$- bis $C_6$-alkyl)diethanolamine, wie Methyldiethanolamin oder N-Alkyl-dialkylentriamin, wie N-Methyldipropylentriamin.

Bei der Komponente Diisocyanat (Komponente c), i) sowie m)) handelt es sich um übliche aliphatische, cycloaliphatische und/oder aromatische Diisocyanate, wie Tetramethylendiisocyanat, Hexamethylendiisocyanat, Methylendiphenyldiisocyanat, 2,4- und 2,6-Toluylendiisocyanat und deren Isomerengemische, o-, m- und p-Xylylendiisocyanat, 1,5-Naphthylendiisocyanat, 1,4-Cyclohexylendiisocyanat, Dicyclohexylmethandiisocyanat und Mischungen davon, insbesondere Isophorondiisocyanat, Hexamethylendiisocyanat und/oder Dicyclohexylmethandiisocyanat. Gewünschtenfalls können bis zu 3 Mol-% der genannten Verbindungen durch Triisocyanate ersetzt sein.

Wie bei der Herstellung von Polyurethanen und Polyharnstoffen üblich, kann man Kettenverlängerer verwenden. Geeignete Kettenverlängerer sind beispielsweise Hexamethylendiamin, Piperazin, 1,2-Diaminocyclohexan, 1,3-Diaminocyclohexan, 1,4-Diaminocyclohexan, Neopentandiamin und 4,4'-Diaminodicyclohexylmethan.

Die beschriebenen Polyurethane und Polyharnstoffe sind vorzugsweise dadurch erhältlich, daß man die Reaktionspartner für die Diisocyanate unter einer Inertgasatmosphäre in einem inerten Lösungsmittel, z.B. Methylethylketon bei Verbindungen mit OH-Gruppen und Wasser oder einem Alkohol wie Ethanol bei Verbindungen mit NH-Gruppen, bei Temperaturen von 30°C bis 110°C, bevorzugt bei 40°C bis 100°C bei Verbindungen mit OH-Gruppen und unter 50°C, bevorzugt bei 5°C bis 30°C bei Verbindungen mit NH-Gruppen mit den Diisocyanaten umsetzt. Diese Umsetzung kann gegebenenfalls in Gegenwart von Kettenverlängerern durchgeführt werden, um Polyurethane bzw. Polyharnstoffe mit höheren Molekulargewichten herzustellen. Die Umsetzung kann durch Zugabe von Katalysatoren wie zinnorganischen Verbindungen, z.B. Dibutylzinndilaurat, Tetraalkyltitanate insbesondere bei Reaktanden mit OH-Gruppen, beschleunigt werden. Wie bei der Herstellung von Polyurethanen üblich, werden die Reaktionspartner für die Diisocyanate und die Diisocyanate selbst zweckmäßigerweise im molaren Verhältnis von 0,8 bis 1,1 : 1 eingesetzt.

Die Reaktion kann bei Polyurethanen ohne Lösungsmittel oder in einem geeigneten inerten Lösungsmittel oder Lösungsmittelgemisch erfolgen. Geeignete Lösungsmittel sind aprotische polare Lösungsmittel, z.B. Tetrahydrofuran, Essigsäureethylester, N-Methylpyrrolidon, Dimethylformamid und bevorzugt Ketone, wie Aceton und Methylethylketon. Vorzugsweise erfolgt die Reaktion unter einer Inertgasatmosphäre, wie z.B. unter Stickstoff. Des Weiteren erfolgt die Reaktion vorzugsweise bei Umgebungsdruck oder unter erhöhtem Druck. Die Komponenten werden bevorzugt in solchen Mengen eingesetzt, daß das Verhältnis von NCO-Äquivalent der Verbindungen des Diisocyanats zu Äquivalent aktives Wasserstoffatom der übrigen Komponenten in einem Bereich von etwa 0,6:1 bis 1,4:1, bevorzugt 0,8:1 bis 1,2:1, insbesondere 0,9:1 bis 1,1:1, liegt. Gegebenenfalls noch vorhandene freie Isocyanatgruppen der Polyurethane können durch anschließende Umsetzung mit Aminen, vorzugsweise Aminoalkoholen, inaktiviert werden. Geeignete Amine und Aminoalkohole sind die zuvor genannten, bevorzugt 2-Amino-2-methyl-1-propanol oder tert.-Aminhaltiges Diamin, wie N,N-Dimethylaminopropyldiamin.

Die beschriebenen Polyurethane und Polyharnstoffe sind aufgrund ihrer ionogenen Gruppierungen, insbesondere beim Vorliegen von Ladungen, in der Regel leicht alkohol- und wasserlöslich oder zumindest ohne Zuhilfenahme von Emulgatoren in Alkohol und Wasser dispergierbar. Als Alkohole sind hier insbesondere kurzkettige $C_1$-$C_4$-Alkanole wie Methanol, Ethanol, iso-Propanol oder n-Propanol gemeint. Geladene kationische Gruppierungen lassen sich aus den vorliegenden tertiären Aminstickstoffatomen entweder durch Protonierung, z.B. mit Phosphorsäure oder Carbonsäuren wie Milchsäure, oder durch Quaternisierung, z.B. mit Alkylierungsmitteln wie $C_1$- bis $C_4$-Alkylhalogeniden oder

-sulfaten in den Polyharnstoffen erzeugen. Beispiele solcher Alkylierungsmittel sind Ethylchlorid, Ethylbromid, Methylchlorid, Methylbromid, Dimethylsulfat und Diethylsulfat. Die anionischen bzw. anionogenen Gruppierungen sind in den entsprechenden Aminen vorhanden. Da ein Teil der beschriebenen Polyurethane und Polyharnstoffe neue Stoffe darstellen, betrifft die vorliegende Erfindung weiterhin diese neuen Stoffe.

Durch Neutralisation anionogener Gruppen unterschiedlicher Grundpolymere A) durch je eine Aminogruppe eines Amins (Neutralisationsmittels) wird zumindest ein Teil der Polymerketten unter Salzbildung vernetzt.

Als Neutralisationsmittel zur (Teil)neutralisation der anionogenen Grundpolymere sind alle unter den Neutralsationsmitteln aufgeführten Amine geeignet, diese können jeweils einzeln oder in Form von Gemischen eingesetzt werden. Zur Neutralisation der anionogenen Gruppen der Grundpolymere können auch Mischungen eingesetzt werden, die mindestens ein Amin sowie mindestens eine weitere Base enthalten. Geeignete weitere Basen für die Neutralisation der Polymere sind Alkalimetallbasen, wie Natroniauge, Kaiiiauge, Natriumcarbonat, Kaliumcarbonat, Natriumhydrogencarbonat, Kaliumhydrogencarbonat und Erdalkalimetallbasen, wie Kalziumhydroxid, Kalziumoxid, Magnesiumhydroxid oder Magnesiumcarbonat sowie Ammoniak und Amine, die kein Gegenion B) ausbilden.

Wird als Grundpolymer ein Polyurethan, Poly(urethan-urea) oder Polyharnstoff eingesetzt, deras zusätzlich Amingruppen als kationogene Gruppen aufweist, so können diese durch Neutralisation mit einer Säure wie Milchsäure oder Phosphorsäure oder durch Quaternisierung teilweise oder vollständig in die entsprechenden kationischen Gruppen überführt werden.

Polyurethane, Poly(urethan-urea) oder Polyharnstoff, die sowohl kationogene als auch anionogene Gruppen aufweisen, können nacheinander einer Neutralisation mit wenigstens einer Säure, einer Neutralisation mit wenigstens einer Base und gewünschtenfalls zusätzlich einer Quaternisierung unterzogen werden. Die Reihenfolge der Neutralisationsschritte ist dabei im Allgemeinen beliebig.

Gewünschtenfalls können kationogene Gruppen auch teilweise oder vollständig quaternisiert werden. Die Quaternisierung kann z.B. mit Alkylierungsmitteln, wie $C_1$- bis $C_4$-Alkylhalogeniden oder Sulfaten erfolgen. Bevorzugte Alkylierungsmittel sind Ethylchlorid, Ethylbromid, Methylchlorid, Methylbromid, Dimethylsulfat und Diethylsulfat.

Bevorzugt beträgt der Anteil des Neutralisationspolymers an dem polymeren Salz mindestens etwa 1 Gew.-%, insbesondere mindestens 3 Gew.-%.

Bevorzugt beträgt der Anteil des Neutralisationspolymers an dem polymeren Salz höchstens etwa 50 Gew.-%, bevorzugt höchstens etwa 40 Gew.-%.

Die erfindungsgemäßen wasserlöslichen oder wasserdispergierbaren polymeren Salze sind erhältlich durch Umsetzung der Grundpolymere, enthaltend an die Polymerkette gebundene anionogene und/oder anionische Gruppen, mit wenigstens einem Neutralisationspolymer. Die Umsetzung kann vorzugsweise im Anschluß an die Herstellung der Grundpolymere und im Allgemeinen im gleichen Reaktionsgefäß erfolgen. Gewünschtenfalls kann zur Herstellung der erfindungsgemäßen Salze auch ein separat hergestelltes oder kommerziell erhältliches Grundpolymer eingesetzt werden. Geeignete Polyurethane werden z.B. in der DE-A-42 41 118, DE-A-42 25 045 und EP-A 0 619 111 beschrieben, auf die in vollem Umfang Bezug genommen wird. Geeignete Acrylatcopolymere werden z.B. in der DE-A-39 01 325 und der DE-A-43 14 305 beschrieben, auf die in vollem Umfang Bezug genommen wird. Geeignete Lösungsmittel für die Umsetzung sind die zuvor bei der Herstellung der Polyurethane genannten.

Die Neutralisation von Säuregruppen enthaltenden Grundpolymeren kann durch Zugabe einer Base oder eines Basengemischs erfoigen.

Wird bei der Herstellung der polymeren Salze ein wassermischbares organisches Lösungsmittel eingesetzt, so kann dieses im Anschluss durch übliche, dem Fachmann bekannte Verfahren, z.B. durch Destillation bei vermindertem Druck, entfernt werden.

Vor dem Abtrennen des Lösungsmittels kann dem polymeren Salz zusätzlich Wasser zugegeben werden. Nach Ersatz des Lösungsmittels durch Wasser erhält man eine Lösung oder Dispersion des polymeren Salzes, aus der, falls gewünscht, das polymere Salz in üblicher Weise gewonnen werden kann, z.B. durch Sprühtrocknung.

Der pH-Wert der wäßrigen Lösungen oder Dispersionen der polymeren Salze kann durch Zugabe einer Säure oder Base eingestellt werden. Geeignete Säuren und Basen sind die zuvor als zusätzliche Neutralisierungsmittel genannten. Vorzugsweise liegt der pH-Wert für anionische polymere Salze im alkalischen Bereich, insbesondere > 7,5. Vorzugsweise liegt der pH-Wert für kationische polymere Salze im sauren Bereich, insbesondere bei 5,5 bis 6,5.

Die erfindungsgemäßen polymeren Salze sind wasserlöslich oder wasserdispergierbar. Sie bilden im Allgemeinen klare und klebfreie Filme und lassen sich mit Wasser sehr gut auswaschen. Vorteilhafterweise werden mit den erfindungsgemäßen polymeren Salzen auch Filme mit einer sehr guten Elastizität erhalten. Diese ist im Allgemeinen höher als die Elastizität, die üblicherweise bei aus dem Stand der Technik bekannten Polyurethanen mit kurzkettigen Neutralisationsmitteln erhalten wird.

Im Rahmen der vorliegenden Erfindung umfassen die Ausdrücke Alkyl und Alkylen geradkettige und verzweigte Alkyl- bzw. Alkylengruppen. Vorzugsweise handelt es sich dabei um geradkettige oder verzweigte, $C_1$- bis $C_{40}$- und besonders bevorzugt $C_2$- bis $C_{30}$-Alkyl- und Alkylengruppen.

$C_1$- bis $C_6$-Alkyl bzw. steht vorzugsweise für Methyl, Ethyl, Isopropyl, n-Butyl, 2-Butyl, sec.-Butyl, tert.-Butyl,

n-Pentyl, 2-Pentyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 2-Hexyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethylbutyl, 1,1,2-Trimethylpropyl, 1,2,2-Trimethylpropyl, 1-Ethylbutyl, 2-Ethylbutyl, 1-Ethyl-2-methylpropyl etc.

$C_2$- bis $C_6$-Alkylen steht für geradkettige und verzweigte $C_2$- bis $C_6$-Alkylenreste, bevorzugt $C_2$- bis $C_4$-Alkylenreste. Dazu zählen vorzugsweise Ethylen, Propylen, Propan-1,3-diyl, Butan-1,4-diyl, Butan-1,3-diyl, Butan-1,2-diyl, Butan-2,3-diyl, 2-Methylpropan-1,3-diyl, Pentan-1,5-diyl, Pentan-1,4-diyl, Pentan-1,3-diyl, Pentan-1,2-diyl, 1-Methylbutan-1,4-diyl, 2-Methylbutan-1,4-diyl, Hexan-1,6-diyl, Hexan-1,5-diyl, Hexan-1,4-diyl, Hexan-1,3-diyl, Hexan-1,2-diyl etc.

$C_6$- bis $C_{40}$-Alkenyl steht vorzugsweise für geradkettige und verzweigte Alkenylgruppen, die einfach, zweifach oder mehrfach ungesättigt sein können. Bevorzugt handelt es sich um Cg- bis $C_{35}$-, insbesondere um $C_{10}$- bis $C_{30}$- und speziell um $C_{12}$- bis $C_{26}$-Alkenylgruppen. Dazu zählen insbesondere Hexenyl, Heptenyl, Octenyl, Nonenyl, Decenyl, Undecenyl, Dodecenyl, Tridecenyl, Tetradecenyl, Pentadecenyl, Hexadecenyl, Heptadecenyl, Octadecenyl, Nonadecenyl, Linolyl, Linolenyl, Elaostearylyl etc.

Bei den anionogenen Gruppen handelt es sich um Carbonsäuregruppen und/oder Sulfonsäuregruppen und/oder Phosphat und/oder Phosphonatgruppen.

Bei den anionischen Gruppen handelt es sich vorzugsweise um Carboxylat- und/oder Sulfonatgruppen. Diese weisen als Gegenion vorzugsweise ein Alkalimetall, insbesondere Na oder K, oder ein Ammoniumion auf, das von Ammoniak oder einem von den als Neutralisationsmittel eingesetzten Aminen verschiedenen primären, sekundären oder tertiären Amin abgeleitet ist.

Bei den kationogenen Gruppen handelt es sich um primäre, sekundäre und besonders tertiäre Amine. Als kationische Gruppen handelt es sich um Protonierungs- und/oder Quaternisierungsprodukt eines Amins und vorzugsweise um das Produkt der Umsetzung mit einer Mineralsäure, wie Salzsäure oder Schwefelsäure, oder das Produkt der Umsetzung mit einem Quaternisierungsmittel. Zur Quaternisierung eignen sich die üblichen Alkylierungsmittel.

Die Quaternierungsmittel führen eine $C_1$-$C_6$-Alkyl, $C_5$-$C_8$-Cycloalkyl, Phenyl, Phenyl-$C_1$-$C_4$-Alkyl ein.

Geeignete N-Vinylamide sind N-Vinylformamid, N-Vinylacetamid, N-Vinylpropionamid etc. Bevorzugt wird N-Vinylformamid eingesetzt.

Geeignete N-Vinyllactame und deren Derivate sind die z.B. einen oder mehrere $C_1$-$C_6$-Alkylsubstituenten, wie Methyl, Ethyl, n-Propyl, Isopropyl, n-Butyl, sec.-Butyl, tert.-Butyl etc. aufweisen können. Dazu zählen z.B. N-Vinylpyrrolidon, N-Vinylpiperidin, N-Vinylcaprolactam, N-Vinyl-5-methyl-2-pyrrolidon, N-Vinyl-5-ethyl-2-pyrrolidon, N-Vinyl-6-methyl-2-piperidin, N-Vinyl-6-ethyl-2-piperidin, N-Vinyl-7-methyl-2-caprolactam, N-Vinyl-7-ethyl-2-caprolactam etc.

Geeignete Amin-haltige Monomere sind beispielsweise die der folgenden Formel

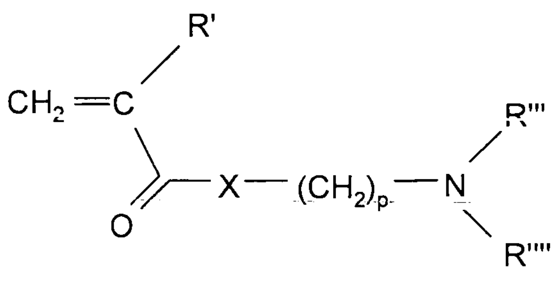

wobei

| | |
|---|---|
| R' | = H, $CH_3$ |
| X | = O, NH |
| R''', R'''' | = gleich oder verschieden sind und für $CH_3$, $-C_2H_5$, $-C_3H_7$, $-C_4H_9$, -tert.-$C_4H_9$ stehen können |
| p | = 1 bis 5 |

Besonders geeignete Monomere sind:

- N,N-Dimethylaminoethyl(meth)acrylat
- N,N-Dimethylaminopropyl(meth)acrylat
- N,N-Dimethylaminoethyl(meth)acrylamid
- N,N-Dimethylaminopropyl(meth)acrylamid.

Ganz besonders geeignete Monomere sind:

- N,N-Dimethylaminoethylmethacrylat
- N,N-Dimethylaminoethylmethacrylamid
- N,N-Dimethylaminopropylmethacrylamid.

Geeignete COOH-haltige Monomere sind beispielsweise α-,β-ethylenisch ungesättigte Mono-und Dicarbonsäuren, wie z.B. Acrylsäure, Methacrylsäure, Fumarsäure, Maleinsäure, Itaconsäure, Crotonsäure und Mischungen davon. Bevorzugt werde Acrylsäure, Methacrylsäure und Mischungen davon eingesetzt.

Die erfindungsgemäßen polymeren Salze sowie die erfindungsgemäßen Polyharnstoffe (zusammengefaßt unter dem Begriff erfindungsgemäße Polymere) eignen sich besonders gut als oder in Haarfestigerpolymer(en), die dem Haar Flexibilität verleihen.

Die erfindungsgemäßen Polymere eignen sich als Hilfsmittel in der Kosmetik. Ein weiterer Gegenstand der Erfindung betrifft daher die Verwendung der erfindungsgemäßen Polymere in kosmetischen Mitteln, insbesondere als Filmbildner und/oder Beschichtungsmittel und/oder Bindemittel. Hierbei ist ihr Einsatz insbesondere in kosmetischen Mittel zur Behandlung von keratinhaltigen und keratinanalogen Oberflächen, wie Haar, Haut und Nägel, brauchbar. Sie eignen sich insbesondere für die Haarkosmetik, vorzugsweise als Festigerpolymer in Haarsprays, Schaumfestigern, Haarmousse, Haargel und Shampoos. Sie eignen sich weiterhin bevorzugt für eine Verwendung in pigmenthaltigen kosmetischen Mitteln, wie beispielsweise in der dekorativen Kosmetik, insbesondere in Mascara und Lidschatten. Auch eignen sie sich zur Herstellung von stiftförmigen kosmetischen Produkte, wie Deostifte, Schminkstifte etc..

Die erfindungsgemäßen Polymere eignen sich weiterhin als Hilfsmittel in der Pharmazie, bevorzugt als oder in Beschichtungsmittel(n) oder Bindemittel(n) für feste Arzneiformen brauchbar. Ein weiterer Gegenstand der Erfindung betrifft daher die Verwendung der erfindungsgemäßen Polymere in pharmazeutischen Mitteln, insbesondere als Filmbildner und/oder Beschichtungsmittel und/oder Bindemittel. So können die zuvor genannten Polymere als Tablettenüberzugmittel und Tablettenbindemittel verwendet werden.

Die erfindungsgemäßen Polymeren eignen sich weiterhin vorzugsweise für die Verwendung als oder in Beschichtungsmittel(n) für die Textil-, Papier-, Druck-, Leder-, und Klebstoffindustrie.

Die erfindungsgemäßen Polymere eignen sich als Bestandteil, als Wirkpolymer, Filmbildner, Beschichtungsmittel, Bindemittel, (Co-)Emulgator, Penetrationsinhibitor, Kristallisationsinhibitor, feuchtigkeitshaltende Additive.

Die erfindungsgemäßen Polymere eignen sich weiterhin als Viskositätseinstellende Mittel. Insbesondere bei Öl- und Wasserhaltigen kosmetischen Mitteln, wie beispielsweise Cremes oder Lotionen kann, in Abhängigkeit von dem jeweiligen Öl- bzw. Wasseranteil durch Zusatz der erfindungsgemäßen Polymere die Viskosität gezielt auf die gewünschte Größe eingestellt werden.

Ein weiterer Gegenstand der Erfindung ist ein kosmetisches und/oder pharmazeutisches Mittel, das wenigstens ein erfindungsgemäßes Polymer (= Polyharnstoff nach Anspruch 1 oder polymeres Salz) enthält. Im Allgemeinen enthält das Mittel die erfindungsgemäßen Polymere in einer Menge im Bereich von etwa 0,2 bis 30 Gew.-%, insbesondere von 0,5 bis 20 Gew.-%, bezogen auf das Gesamtgewicht des Mittels.

Sind die in den erfindungsgemäßen Mitteln eingesetzten erfindungsgemäßen polymeren Salze wasserdispergierbar, können sie in Form von wäßrigen Dispersionen mit Teilchendurchmessern von üblicherweise 1 bis 350 nm, bevorzugt 1 bis 250 nm, zur Anwendung gebracht werden. Die Feststoffgehalte der Präparate liegen dabei üblicherweise in einem Bereich von etwa 0,2 bis 30, insbesondere 0,5 bis 20 Gew.-%, bevorzugt 1 bis 12 Gew.-%. Diese Mikrodispersionen benötigen in der Regel keine Emulgatoren oder Tenside zu ihrer Stabilisierung.

Bevorzugt können die erfindungsgemäßen Mittel in Form eines Haarbehandlungsmittels, wie Schaumfestiger, haarmousse, Haargel, Shampoo und insbesondere in Form eines Haarsprays vorliegen. Zur Verwendung als Konditionspolymer oder Festigerpolymer sind dabei Mittel bevorzugt, welche die erfindungsgemäßen Ppolymere polymeren Salze enthalten, die wenigstens eine Glasübergangstemperatur $T_g \geq 10°C$, bevorzugt $\geq 20°C$, aufweisen. Der K-Wert dieser Polymere (gemessen nach E. Fikentscher, Cellulose-Chemie 13 (1932), S. 58-64) an einer 1 gew.-%igen Lösung in N-Methylpyrrolidon, liegt vorzugsweise in einem Bereich von 23 bis 90, insbesondere 25 bis 60.

Weisen die erfindungsgemäßen Polymere Salze Siloxangruppen auf, so beträgt der Siloxangehalt dieser Polymere im Allgemeinen 0,05 bis 20 Gew.-%, bezogen auf das Gesamtgewicht der eingebauten Komponenten.

Bei den erfindungsgemäßen kosmetischen Mitteln handelt es sich vorzugsweise um Haarbehandlungsmittel. Diese liegen üblicherweise in Form einer wäßrigen Dispersion oder in Form einer alkoholischen oder wäßrig-alkoholischen Lösung vor. Beispiele geeigneter Alkohole sind Ethanol, Propanol, Isopropanol etc.

Weiter können die erfindungsgemäßen Haarbehandlungsmittel im Allgemeinen übliche kosmetische Hilfsstoffe enthalten, beispielsweise Weichmacher, wie Glycerin und Glykol; Emollienzien; Parfüms; Tenside; UV-Absorber; Farbstoffe; antistatische Mittel; Mittel zur Verbesserung der Kämmbarkeit; Konservierungsmittel; und Entschäumer.

Wenn die erfindungsgemäßen kosmetischen Mittel als Haarspray formuliert sind, enthalten sie eine ausreichende Menge eines Treibmittels, beispielsweise einen niedrigsiedenden Kohlenwasserstoff oder Ether, wie Propan, Butan, Isobutan oder Dimethylether. Als Treibmittel sind auch komprimierte Gase brauchbar, wie Stickstoff, Luft oder Kohlendioxid. Die Menge an Treibmittel kann dabei gering gehalten werden, um den VOC-Gehalt nicht unnötig zu

erhöhen. Sie beträgt dann im Allgemeinen nicht mehr als 55 Gew.-%, bezogen auf das Gesamtgewicht des Mittels. Gewünschtenfalls sind aber auch höhere VOC-Gehalte von 85 Gew.-% und darüber möglich.

Die erfindungsgemäßen Polymere können auch in Kombination mit anderen Polymeren in den Mitteln zur Anwendung kommen. Die erfindungsgemäßen Polymere eigenen sich somit auch zur Verbesserung der Elastizität herkömmlicher Haarbehandlungsmittel, insbesondere Haarfestigungsmittel. Diese verleihen den Haaren dann im Allgemeinen eine sehr gute Flexibilität und Geschmeidigkeit.

Solche anderen Polymere sind insbesondere:

- nicht-ionische, wasserlösliche bzw. wasserdispergierbare Polymere oder Oligomere, wie Polyvinylcaprolactam, z.B. Luviskol Plus (BASF), oder Polyvinylpyrrolidon und deren Copolymere, insbesondere mit Vinylestern, wie Vinylacetat, z.B. Luviskol VA 37 (BASF); Polyamide, z.B. auf Basis von Itaconsäure und aliphatischen Diaminen;
- amphotere oder zwitterionische Polymere, wie die unter den Bezeichnungen Amphomer® (National Starch) erhältlichen Octylacrylamid/Methylmethacrylat/tert.-Butylaminoethylmethacrylat/2-Hydroxypropylmethacrylat-Copolymere sowie zwitterionische Polymere, wie sie beispielsweise in den deutschen Patentanmeldungen DE 39 29 973, DE 21 50 557, DE 28 17 369 und DE 37 08 451 offenbart sind. Acrylamidopropyltrimethylammoniumchlorid/Acrylsäure- bzw. -Methacrylsäure-Copolymerisate und deren Alkali- und Ammoniumsalze sind bevorzugte zwitterionische Polymere. Weiterhin geeignete zwitterionische Polymere sind Methacroylethylbetain/Methacrylat-Copolymere, die unter der Bezeichnung AmersetteR (AMERCHOL) im Handel erhältlich sind, und Copolymere aus Hydroxyethylmethacrylat, Methylmethacrylat, N,N-Dimethylaminoethylmethacrylat und Acrylsäure (Jordapon®);
- anionische Polymere, wie Vinylacetat/Crotonsäure-Copolymere, wie sie beispielsweise unter den Bezeichnungen Resyn® (NATIONAL STARCH), Luviset® (BASF) und Gafset® (GAF) im Handel sind, Vinylpyrrolidon/Vinylacrylat-Copolymere, erhältlich beispielsweise unter dem Warenzeichen Luviflex® (BASF). Ein bevorzugtes Polymer ist das unter der Bezeichnung Luviflex®VBM-35 (BASF) erhältliche Vinylpyrrolidon/Acrylat-Terpolymer. Acrylsäure/Ethylacrylat/N-tert.Butylacrylamid-Terpolymere, die beispielsweise unter der Bezeichnung Ultrahold® strong (BASF) vertrieben werden, sowie Luvimer® (BASF, Terpolymer aus t-Butylacrylat, Ethylacrylat und Meth- acrylsäure), Natriumsulfonat-haltige Amide oder Natriumsulfonat-haltige Polyester, oder
- kationische (quaternisierte) Polymere, z.B. kationische Polyacrylatcopolymere auf Basis von N-Vinyllactamen und deren Derivaten (N-Vinylpyrrolidon, N-Vinylcaprolactam etc.) sowie übliche kationische Haarconditionerpolymere, z.B. Luviquat® (Copolymer aus Vinylpyrrolidon und Vinylimidazoliummethochlorid), Luviquat® Hold (Copolymerisat aus quaternisiertem N-Vinylimidazol, N-Vinylpyrrolidon und N-Vinylcaprolactam), Merquat® (Polymer auf Basis von Dimethyldiallylammoniumchlorid), Gafquat® (quaternäre Polymere, die durch Reaktion von Polyvinylpyrrolidon mit quaternären Ammoniumverbindungen entstehen), Polymer JR (Hydroxyethylcellulose mit kationischen Gruppen), Polyquaternium-Typen (CTFA-Bezeichnungen) etc.;
- nichtionische, siloxanhaltige, wasserlösliche oder -dispergierbare Polymere, z.B. Polyethersiloxane, wie Tegopren® (Fa. Goldschmidt) oder Belsil® (Fa. Wacker).

Die erfindungsgemäßen Polymere können als Mischung mit einem amidgruppenhaltigen Polymer eingesetzt werden. Dazu zählen z.B. die in der DE-A-42 25 045 beschriebenen Polyurethane, die zuvor beschriebenen Vinylpyrrolidon/Acrylat-Terpolymere und Acrylsäure/Ethylacrylat/N-tert.-Butylacrylamid-Terpolymere (z.B. Ultrahold® strong der BASF AG), die zuvor beschriebenen amidgruppenhaltigen amphoteren Polymere (z.B. Amphomer®) und insbesondere Copolymerisate, die einen Anteil an amidgruppenhaltigen Monomeren, wie N-Vinyllactamen, von mindestens 30 Gew.-% aufweisen (z.B. Luviskol®plus und Luviskol®VA37 der BASF AG).

Die anderen Polymere sind vorzugsweise in Mengen bis zu 10 Gew.-%, bezogen auf das Gesamtgewicht des Mittels enthalten.

Ein bevorzugtes Haarbehandlungsmittel enthält:

- 0,2 bis 30, insbesondere 0,5 bis 20 Gew.-%, bevorzugt 1 bis 10 Gew.-%, mindestens eines erfindungsgemäßen Polymers (= Polyharnstoff nach Anspruch 1 sowie polymeren Salzes nach Anspruch 5),

- 50 bis 99,5 Gew.-%, bevorzugt 55 bis 99 Gew.-%, eines Lösungsmittels, bevorzugt ausgewählt unter Wasser und wassermischbarens Lösungsmitteln, bevorzugt $C_2$- bis $C_5$-Alkoholen, insbesondere Ethanol, und Mischungen davon,

- 0 bis 70 Gew.-%, bevorzugt 0,1 bis 50 Gew.-%, eines Treibmittels,
  Als Treibmittel wird üblicherweise Propan/Butan, vorzugsweise Dimethylether, eingesetzt,

- 0 bis 10 Gew.-%, bevorzugt 0,1 bis 10 Gew.-%, mindestens eines von den erfindungsgemäßen Polymeren verschiedenes, in Wasser löslichen oder dispergierbaren Polymers,

- 0 bis 0,5 Gew.-%, bevorzugt 0,001 bis 2 Gew.-%, mindestens einer wasserlöslichen oder wasserdispergierbaren Siliconverbindung.
  Das erfindungsgemäße Mittel kann als Siliconverbindung mindestens ein nichtionisches, siloxanhaltiges, wasserlösliches oder -dispergierbares Polymer, insbesondere ausgewählt unter den zuvor beschriebenen Polyethersiloxanen, enthalten. Der Anteil dieser Komponente beträgt dann im Allgemeinen etwa 0,001 bis 2 Gew.-%, bezogen auf das Gesamtgewicht des Mittels.

Das erfindungsgemäße Mittel kann als zusätzliche Komponente mindestens ein wasserunlösliches Silicon, insbesondere ein Polydimethylsiloxan, z.B. die Abil®-Typen der Fa. Goldschmidt, enthalten. Der Anteil dieser Komponente beträgt dann im Allgemeinen etwa 0,0001 bis 0,2 Gew.-%, bevorzugt 0,001 bis 0,1 Gew.-%, bezogen auf das Gesamtgewicht des Mittels.

Das erfindungsgemäße Mittel kann zusätzlich gegebenenfalls einen Entschäumer, z.B. auf Silicon-Basis, enthalten. Die Menge des Entschäumers beträgt im Allgemeinen bis zu etwa 0,001 Gew.-%, bezogen auf die Gesamtmenge des Mittels.

Das erfindungsgemäße Mittel enthält vorzugsweise zusätzlich zu den zuvor genannten Komponenten:

- 0 bis 40 Gew.-%, bevorzugt 0,1 bis 35 Gew.-%, wenigstens eines Tensids,

- 0 bis 5,0 Gew.-% eines Emulgators,

- 0 bis 3 Gew.-%, bevorzugt 0,05 bis 2,5 Gew.-%, wenigstens eines Salzes,

- 0 bis 3 Gew.-%, bevorzugt 0,05 bis 2,5 Gew.-%, wenigstens eines Verdickers, sowie gegebenenfalls weitere übliche Zusatzstoffe. Als weitere Zusatzstoffe seien genannt: Farbstoffe, Pigmente, UV-Absorber (die jeweils in Mengen von 0 bis 5,0 Gew.-% eingesetzt werden) sowie Konservierungsmittel und Parfümstoffe. Diese liegen dann im Allgemeinen jeweils in einer Menge von etwa 0 bis 0,2 Gew.-%, bevorzugt 0,001 bis 0,2 Gew.-% vor.
  Alle genannten Gewichtsangaben beziehen sich auf das Gesamtgewicht des Mittels.
  Die erfindungsgemäßen Mittel besitzen den Vorteil, daß sie einerseits den Haaren die gewünschte Festigkeit verleihen und die Polymere leicht auswaschbar (redispergierbar) sind und das Haar andererseits elastisch, geschmeidig und klebfrei bleibt.
  Die Erfindung wird anhand der folgenden, nicht einschränkenden Beispiele näher erläutert.

Beispiele

Die Beurteilung der Flexibilität der Polymere wurde wie folgt bestimmt:

Aus 20%iger Polymerlösung mit einer Schichtdicke von 90 bis 140 mm werden 2 bis 5 mm breite Streifen ausgestanzt und bezüglich Griff, Festigkeit und Flexibilität getestet. Bei der Prüfung von Klebrigkeit, Klarheit und Auswaschbarkeit wird zur Testung eine Glasplatte mit den Filmen beschichtet.
Für die Bewertung werden

- entweder aus einer 20%-igen ethanolischen. Lösung auf PE-Foile 500 mmm-Film gerackelt und getrocknet.

  - Elastizität: Spröde (4)/hart (3)/mäßig (2)/sehr weich (1)
  - Zug-Festigkeit: verfliessen (4)/schlecht (3)/mäßig (2)/fest (1)
  - Griff: rauh, hemmend (4)/hemmend (3)/gut (2)/geschmeidig-glatt (1)

- oder aus einer 5%-igen wäßrigen ethanolischen Lösung auf Glasplatte Filme beschichten

  - Klebrigkeit: klebrig (4)/leicht klebrig (3)/befriedigen (2)/nicht klebrig (1)
  - Auswaschbarkeit: nicht löslich (4)/schlecht (3)/befriedigen (2)/klar (1)

Beispiele 1 bis 4

Herstellung von Neutralisationspolymeren

Das Neutralisationspolymer wird entweder aus Diol-Komponenten/Diisocyanat in Methylethylketon oder Aceton/ unter Druck bei 80°C ca. 5 h, oder aus Diamin/Diisocyanat bei einer Temperatur von 10 bis 40°C in Ethanol

polymerisiert.

Beispiel:

| | PEG1500 | A-Si2122 | PEG-DA | MDPTA | Hex-DA | IPDI |
|---|---|---|---|---|---|---|
| 1 | 3 | - | - | 1 | 1.2 | 5 |
| 2 | 3 | 0.5 | - | 1 | 0.7 | 5 |
| 3 | - | 0.5 | 3 | 1 | 0.7 | 5 |
| 4 | - | - | 3 | 1 | 1.2 | 5 |

PEG 1500: Polyethylenglykol, Mn ≈ 1500 (BASF)
A-Si 2122: P(Dimethylsiloxan-diamin), Mn ≈ 900 (Tegomer, Fa. Goldschmidt)
PEG-DA: O,O'-Bis(2-aminopropyl)polyethylenglykol 800; Mn ≈ 900 (Fluka)
MDPTA; N-Methyldipropylentriamin
Hex-DA: Hexandiamin (1,6)
IPDI: Isophorondiisocyanat

Beispiele 5 bis 9

Kationisches Neutralisationspolymer auf Basis von Polyurethan (PUR) für COOH-haltige Polymere:

Allgemeine Herstellungsvorschriften:

Für Beispiel 7:

In einem 4-Halskolben, der unter $N_2$-Atmosphäre mit Rührer, Tropftrichter, Thermometer und Rückflußkühler ausgestattet war, wurden 88,9 g (0,4 Mol) Isophorondiisocyanat in 20 g Ethanol vorgelegt. Bei einer Temperatur von ca. 10°C wurde unter Rühren und Eiskühlung eine Mischung aus 90 g (0,1 Mol) Tegomer A-Si 2122 ($M_n$ = 900 g/Mol) und 30 g Ethanol bei einer Temperatur ≤ 40°C zugetropft. Die Reaktion wurde unter Rühren noch 30 Minuten bei etwa 30 bis 40°C gehalten. Nach dem Abkühlen auf ca. 10°C wurde eine Mischung aus 70 g (0,3 Mol) 0,0'-Bis-(2-aminopropyl) polyetherglykol 800; Mn ≈ 900 (Fluka) und 2,9 g (0,02 Mol) N-Methyldipropylentriamin in 50 g Ethanol zugegeben, wobei die Reaktionstemperatur auf ca. 45°C anstieg. Anschließend verdünnte man das Reaktionsgemisch mit 600 g Ethanol. Man erhielt nach dem Filtrieren eine klare hellgelbe ca. 40%ige ethanolische Polymerlösung.

Analog wurden die Neutralisationspolymere der Beispiele 5, 6, 8, 9 sowie 12 und 13 hergestellt.

| Bsp. | Neutralisationspolymer | PEG-DA (mol) | MDPT A (mol) | A-Si2122 (mol) | $SO_3$NA-PPG-DA (mol) | MAN 0078 (mol) | IPDI (mol) | freie AZ |
|---|---|---|---|---|---|---|---|---|
| 5 | A | 3.5 | 1 | - | - | - | 4 | ca.20 |
| 6 | B | 4.0 | - | 0.5 | - | - | 4 | 11.4 |
| 7 | C | 3.0 | 0.2 | 1 | - | - | 4 | 7.5 |
| 8 | D | 3.0 | - | 1 | 0.2 | - | 4 | 4.8 |
| 9 | E | 3.5 | 2-3 | - | - | 0.2 | 6 | 10 |

PEG-DA: O,O'Bis(2-amino-propyl)polyethylenglykol 800; Mn ≈ 900 (Fluka)
MDPTA: N-Methyl-dipropylentriamin
A-Si 2122: P(Dimethylsiloxan diamin), Mn ≈ 900 (Tegomer, Fa. Goldschmidt)
$SO_3$Na-PPG-DA: α,ω-Polypropylenglykeldiamin, sulfopropyliert; Mn≈ 700 (Raschig)
MAN 00078: Poly(aminopropyl)dimethylsiloxan (Fa. Hüls Silicone) Amin-Zahl von ca. 28 (entspricht Diamin mit Molekulargewidcht von ca. 4000)

AZ: Amin-Zahl
IPDI: Isophorondiisocyanat

Beispiel 10

Kationisches Neutralisationspolymer auf Basis von Polyacrylaten (PA) für COOH-haltige Polymere:

Allgemeine Herstellungsvorschriften:

Für Beispiel 10:

| | | |
|---|---|---|
| Zulauf 1: | Vinylpyrrolidon | 59,5 g |
| | Vinylcaprolactam | 102,0 g |
| | Dimethylaminopropylmethacrylamid | 8,5 g |
| Zulauf 2: | Ethanol | 80,0 g |
| | t-Butylperpivalat | 0,4 g |
| Zulauf 3: | Ethanol | 80,0 g |
| | t-Butylperpivalat | 1,6 g |
| Vorlage: | Ethanol | 95,0 g |
| | Zulauf 1 | 32,0 g |
| | Zulauf 2 | 10,0 g |

Fahrweise:

Vorlage unter $N_2$ in einer Rührapparatur mit 3 Zulaufeinheiten unter Rührung auf 80°C aufheizen. Dann Zulauf 1 und 2 starten. Zulauf 1 in 3 h, Zulauf 2 in 4 h zudosieren. 5h bei 80°C nachpolymerisieren. Zulauf 3 starten und in 1 h bei 80°C zugeben, 5 h bei 80°C nachpolymerisieren. Eine farblose 40%ige ethanolische Polyacrylat-Lösung wurde erhalten.

Analog wurde das Neutralisationspolymer 11 hergestellt.

| Bsp. | PNeutralisationspolymer | VP | VCap | nBA | DMA PMA | AZ |
|---|---|---|---|---|---|---|
| 10 | F | 35 | 60 | - | 5 | 16,5 |
| 11 | G | 55 | - | 40 | 5 | 16,5 |

VP: Vinylpyrrolidon
VCap: Vinylcaprolactam
DMAPMA: Dimethylamino-propylmethacrylamid
nBA: n-Butylacrtylat

Beispiel 12 und 13

Anionisches Neutralisationspolymer auf Basis von Polyurethan (PUR) für aminogruppen-haltige Polymere:

| Bsp. | PNeutralisationspolymer | PEG-DA | A-Si 2122 | DMP A | IPDI (mol) | freie SZ |
|---|---|---|---|---|---|---|
| 12 | H | 3,0 | - | 1 | 4 | 15 |

Tabelle fortgesetzt

| Bsp. | PNeutralisationspolymer | PEG-DA | A-Si 2122 | DMP A | IPDI (mol) | freie SZ |
|---|---|---|---|---|---|---|
| 13 | J | 2,5 | 0,5 | 1 | 4 | 15 |

A-Si 2122: P(Dimethylsiloxan-diamin), Mn ≈ 900 (Tegomer, Fa. Goldschmidt)
PEG-DA: 0,0'-Bis(2-amino-propyl)polyethylenglykol 800; Mn ≈ 900 (Fluka)
SZ: Säure-Zahl
DMPA: Dimethylolpropansäure
IPDI: Isophorondüsocyanat

Beispiele 14 bis 16

Kationische Neutralisationspolymere

| Bsp. | Neutralisationspolymer | VP | nBA | TBA | DMAP MA | MAS | Belsil 6031 | AZ |
|---|---|---|---|---|---|---|---|---|
| 14 | K | 70 | - | 21 | 6 | 3 | - | 19,8 |
| 15 | L | - | 90 | - | 5 | 5 | - | 16,5 |
| 16 | M | - | - | 70 | 3 | 22 | 5 | 9,9 |

TBA: t-Butylacrylat
NAS: Methacrylsäure
Belsil 6031: wasserlösliches ethoxiliertes Silikontensid (Wacker Belsil DMC 6031)

Beispiel 17

Herstellung von COOH-haltigem Polyurethan $PUR^{\ominus}$ (Grundpolymer I)

PUR aus Polyesterdiol/Neopentylglykol/Dimethylolpropansäure/ Isophorondiisocyanat

In einem 4-Halskolben, der mit Rührer, Tropftrichter, Thermometer, Rückflußkühler und einer Vorrichtung für das Arbeiten unter Stickstoff ausgestattet war, wurden 500 g [0,5 Mol] Polyesterdiol aus Isophtalsäure/Adipinsäure und Hexandiol 1,6 (Mw = 1000 g/mol), 201 g [1,5 Mol] Dimethylolpropansäure (DMPA) und 104 g [1 Mol] Neopentylglykol (NPG) in 370 g Methylethylketon unter Erhitzen auf einer Temperatur von 80°C und unter Rühren gelöst. Sobald sich alles gelöst hatte, wurde das Reaktionsgemisch auf ca. 50°C abgekühlt. Anschließend wurden unter Rühren 699,3 g [3,15 Mol] Isophorondiisocyanat zugetropft, wobei die Reaktionstemperatur anstieg. Unter Rückfluß wurde das Reaktionsgemisch dann solange gerührt, bis der NCO-Gehalt des Gemisches praktisch konstant blieb. Danach kühlte man das Gemisch auf RT ab. Das Reaktionsprodukt wurde mit 132,6 g [1,48 Mol] 2-Amino-2-methyl-1-propanol (AMP)/ Wasser bei einer Temperatur ≤ 40°C abgestoppt und zu 90 % neutralisiert. Das Lösungsmittel wurde dann im Vakuum bei 40°C abdestilliert, wobei man eine wäßrige Dispersion erhielt. Pulverförmige Polyurethane können durch Sprühtrockner erhalten werden.

Beispiel 18

Herstellung von COOH-haltigem Polyurethan $PUR^{\ominus}$ (Grundpolymer II)

(PUR aus Polyesterdiol/Neopentylglykol/Dimethylolpropansäure/Isophorondiisocyanat/Hexamethylendiisocyanat)

In einem 4-Halskolben, der mit Rührer, Tropftrichter, Thermometer, Rückflußkühler und einer Vorrichtung für das Arbeiten unter Stickstoff ausgestattet war, wurden 500 g [0,5 Mol] Polyesterdiol aus Isophthalsäure/Adipinsäure und Hexandiol 1,6 (Mw = 1000 g/mol), 201 g [1,5 Mol] Dimethylol-propansäure (DMPA) und 104 g [1 Mol] Neopentylglykol (NPG) in 370g Methylethylketon unter Erhitzen auf einer Temperatur von 80°C und unter Rühren gelöst. Sobald sich alles gelöst hatte, wurde das Reaktionsgemisch auf ca. 50°C abgekühlt. Anschließend wurden unter Rühren ein Gemisch

aus 444 g [2 Mol] Isophorondiisocyanat und 193,4 g [1,15 Mol] Hexamethylendiisocyanat zugetropft, wobei die Reaktionstemperatur anstieg. Unter Rückfluß wurde das Reaktionsgemisch dann solang gerührt, bis der NCO-Gehalt des Gemisches praktisch konstant blieb. Danach kühlte man das Gemisch auf RT ab.

Das Reaktionsprodukt wurde mit 132,6 g [1,48 Mol] 2-Amino-2-methyl-1-propanol (AMP)/Wasser bei einer Temperatur ≤ 40°C abgestoppt und zu 90 % neutralisiert.

Das Lösungsmittel wurde dann im Vakuum bei 40°C abdestilliert, wobei man eine wäßrige Dispersion erhielt. Pulverförmige Polyurethane können durch Sprühtrockner erhalten werden.

Beispiel 19

Herstellung von Polyacrylat aus n-BA/TBA/MAS (40/35/25) (Grundpolymer III)

| | | |
|---|---|---|
| Zulauf 1: | n-Butylacrylat (nBA) | 120,0 g |
| | t-Butylacrylat (TBA) | 105,0 g |
| | Methacrylsäure (MAS) | 75,0 g |
| Zulauf 2: | Ethanol | 100,0 g |
| | t-Butylperpivalat. | 0,9 g |
| Vorlage: | Zulauf 1 | 32,0 g |
| | Zulauf 2 | 10,0 g |
| Zulauf 3: | Ethanol | 150,0 g |
| | t-Butylperpivalat. | 1,6 g |
| Zulauf 4: | Ethanol | 200,0 g |

Fahrweise: Vorlage unter $N_2$ in einer Rührapparatur mit 4 Zulaufeinheiten unter Rührung auf 80°C aufheizen. Dann Zulauf 1 und 2 starten, Zulauf 1 in 3 h Zulauf 2 in 4 h zudossieren. 2 h bei 80°C nachpolymerisieren. Zulauf 3 starten und in 1 h bei 80°C zugeben, 5 h bei 80°C nachpolymeriesieren. Beim Abkühlen mit Zulauf 4 verdünnen.

Beispiel 20

Herstellung von COOH-haltigem Polyacrylat $PA^{\ominus}$ (aus tert.-Butylacrylat und Methacrylsäure 75:25)

Dieses Produkt kann nach der allg. Herstellungsvorschrift von Besipiel 10 hergestellt werden.

Beispiel 21

Herstellung von Amin-haltigem Polyacrylat $PA^{\oplus}$ (aus tert.-Butylacrylat, Vinylpyrrolidon und DMAPMA (Dimethylaminopropylmethacrylamid) (40:45:15)

Dieses Produkt kann nach der alig. Herstellungsvorschrift von Beispiel 10 hergestellt werden.

Beispiel 22

Vergleichsbeispiele V1 bis V5:

| Nr. | | Elatizitätsnote | Zug-Festigkeit | Griff | Klebrigkeit | Auswaschbar | Klarheit |
|---|---|---|---|---|---|---|---|
| V1 | Grundpolymer (I) PU mit AMP neutral. | 3 | 2 | 2 | 1-2 | 1-2 | 1 |
| V2 | Grundpolymer (II) PU mit AMP neutral. | 2 | 2-3 | 2-3 | 2-3 | 1 | 1 |

Tabelle fortgesetzt

| Nr. | | Elatizitätsnote | Zug-Festigkeit | Griff | Klebrigkeit | Auswaschbar | Klarheit |
|---|---|---|---|---|---|---|---|
| V3 | Grundpolymer (III) PA mit AMP neutral. | 2-3 | 2-3 | 2 | 2-3 | 1 | 1 |
| V4 | Neutral.-polymer (C) | 2 | 3 | 1-2 | 3-4 | 2 | 1 |
| V5 | Neutral.-polymer (G) | 2-3 | 2-3 | 2 | 3 | 1-2 | 1-2 |

Erfindungsgemäße Beispiele

| Nr. *) | Grundpolymer: Neutralisationspolymer [Gewicht-Verhältnis] | Elatizitätsnote | Zug-Festigkeit | Griff | Klebrigkeit | Auswaschbar | Klarheit |
|---|---|---|---|---|---|---|---|
| 1 | (I):(A)=[9:1] | 2 | 1-2 | 2 | 1 | 1-2 | 1-2 |
| 2 | (I):(B)=[9:1] | 1-2 | 2 | 1 | 1 | 1-2 | 1 |
| 3 | (I):(C)=[9:1] | 1-2 | 2 | 1 | 1 | 1-2 | 1 |
| 4 | (I):(C)=[8:2] | 1 | 2 | 1 | 1-2 | 1 | 1 |
| 5 | (I):(C)=[7:3] | 1 | 2-3 | 1 | 2 | 1 | 1 |
| 6 | (I):(D)=[9:1] | 2 | 2 | 1-2 | 1 | 1 | 1 |
| 7 | (I):(E)=[9:1] | 1-2 | 1-2 | 1 | 1 | 1-2 | 1 |
| 8 | (11):(A) = [9:1] | 1-2 | 2 | 2 | 2 | 1 | 1-2 |
| 9 | (II):(B)=[9:1] | 1 | 2-3 | 1-2 | 1-2 | 1 | 1 |
| 10 | (II):(C)=[9:1] | 1 | 2 | 1-2 | 1-2 | 1 | 1 |
| 11 | (II):(D)=[9:1] | 1-2 | 2 | 1-2 | 1-2 | 1 | 1 |
| 12 | (II):(E)=[9:1] | 1 | 2 | 1 | 1-2 | 1 | 1 |
| 13 | (III):(A)=[9:1] | 2 | 2 | 2 | 2 | 1 | 1-2 |
| 14 | (III):(B)=[9:1] | 2 | 2-3 | 1-2 | 1-2 | 1 | 1 |
| 15 | (III):(C)=[9:1] | 2 | 2-3 | 1-2 | 1-2 | 1 | 1 |
| 16 | (III):(D)=[9:1] | 2 | 2-3 | 1-2 | 1-2 | 1 | 1-2 |
| 17 | (III):(E)=[9:1] | 2 | 2 | 1-2 | 1-2 | 1 | 1 |
| 18 | (I):(F)=[7:3] | 2-3 | 2 | 1-2 | 2 | 1 | 1 |
| 19 | (I):(G)=[7:3] | 2-3 | 2 | 2 | 1-2 | 1 | 1 |
| 20 | (II):(F)=[8:2] | 2 | 2-3 | 2 | 2 | 1 | 1 |
| 21 | (II):(G)=[8:2] | 2 | 2-3 | 2 | 1-2 | 1 | 1 |
| 22 | (II): (G)= [6:4] | 1-2 | 2-3 | 2 | 1-2 | 1 | 1 |
| 22a | (I): (K) =[5:5] | 3 | 2 | 1-2 | 1-2 | 1 | 1 |
| 22b | (II): (K) =[5:5] | 2 | 2 | 1-2 | 1-2 | | 1 |
| 22c | (II):(K)=[4:6] | 2 | 2 | 1-2 | 1-2 | 1 | 1 |

Tabelle fortgesetzt

| Nr. *) | Grundpolymer: Neutralisationspolymer [Gewicht-Verhältnis] | Elatizitätsnote | Zug-Festigkeit | Griff | Klebrigkeit | Auswaschbar | Klarheit |
|---|---|---|---|---|---|---|---|
| 22d | (II): (L) = [5:5] | 1-2 | 2 | 1-2 | 1-2 | 2-3 | 2 |
| 22e | (II):(M)=[7:3] | 1-2 | 2 | 1-2 | 1-2 | 1-2 | 1 |
| *) : Alle Produkte wurden mit Neutralisationspolymer und Amino-2-methylpropanol bis pH 8 bis 9 neutralisiert | | | | | | | |

Beispiele 23 bis 25

Herstellung von polymeren Salzen

| | | neutralisiert mit | | | | | | |
|---|---|---|---|---|---|---|---|---|
| | | Milchsäure (NG%) | Gewichtsverhältnis*) | Gewichtsverhältnis**) | Flexibilität | Festigung | Curl-Retent. | Aus-waschbarkeit |
| V | PA | 100 | – | – | 3–4 | 1 | 78 | 1 |
| 23 | PA | 90 | 9:1 | | 2–3 | 1 | 75 | 1 |
| 24 | PA | 90 | | 9:1 | 2–3 | 1 | 77 | 1 |
| 25 | PA | 90 | | 8:2 | 2 | 1–2 | 74 | 1 |

V Vergleichsbeispiel: Grundpolymer neutralisiert nur mit Neutralisationsmittel Milchsäure

$PA^{\oplus}$: Polyacrylat aus tert.Butylacrylat, Vinylpyrrolidon und Dimethylamino–propylmethacrylamid 40:45:15 = Grundpolymer nach Beispiel 21

NG: Neutralisationsgrad

*) Grundpolymer: Neutralisationspolymer (H)

**) Grundpolymer: Neutralisationspolymer (J)

Beispiel 20

Verwendung als Haarfestigerpolymer

| Aerosol-Haarspray | [%] |
|---|---|
| Polymer Nr. 1-22 | 3,00 |
| Dimethylether | 40,00 |
| Ethanol | 57,00 |
| Weiterer Zusatz: Silikon, Parfüm, Entschäumer ... | |

| Aerosol-Haarspray | [%] |
|---|---|
| Polymer Nr. 13-22 | 3,00 |
| Propan/Butan-Gas | 40,00 |
| Ethanol | 57,00 |
| Weiterer Zusatz: Silikon, Parfüm, Entschäumer ... | |

| VOC 80 Aerosol-Haarspray | [%] |
|---|---|
| Polymer Nr. 1-22 | 5,00 |
| Wasser | 15,00 |
| Dimethylether | 40,00 |
| Ethanol | 40,00 |
| Weiterer Zusatz: Silikon, Parfüm, Entschäumer ... | |

| VOC 55 Aerosol-Haarspray | [%] |
|---|---|
| Polymer Nr. 1-12 und 18-22 und 22a-e | 3,00 |
| Wasser | 42,00 |
| Dimethylether | 35,00 |
| Ethanol | 20,00 |
| Weiterer Zusatz: Silikon, Parfüm, Entschäumer ... | |

| VOC 55 Handpumpe-Spray | [%] |
|---|---|
| Polymer Nr. 1-12 und 18-22 und 22a-e | 5,00 |
| Wasser | 40,00 |
| Ethanol | 55,00 |
| Weiterer Zusatz: Silikon, Parfüm, Entschäumer ... | |

| Schaumfestiger (= Mousse) | [%] |
|---|---|
| Polymer Nr. 8-22 und 22a-e (25%ige wäßrige Lösung) | 20,00 |

Tabelle fortgesetzt

| Schaumfestiger (= Mousse) | [%] |
|---|---|
| Cremophor A 25 (Ceteareth 25/BASF) | 0,20 |
| Comperlan KD (Cocamide DEA/Henkel) | 0,10 |
| Wasser | 69,70 |
| Propan/Butan | 10,00 |
| Weiterer Zusatz: Parfüm, Konservierungsmittel ... | |

Herstellung: Einwiegen und unter Rühren lösen. Abfüllen und Treibgas zusetzen.

| | Conditioner Shampoo | [%] |
|---|---|---|
| A) | Texapon NSO 28%ig (Sodium Laureth Sulphate/ Henkel) | 50,00 |
| | Comperlan KD (Cocamide DEA / Henkel) | 1,00 |
| | Polymer 22, 22a-e (25%ige wäßrige Lösung) | 20,00 |
| | q.s. Parfümöl | |
| B) | Wasser | 27,50 |
| | Natriumchlorid | 1,50 |
| | q.s. Konservierungsmittel ... | |

Herstellung: Einwiegen und unter Rühren Phasen A und B getrennt lösen und mischen. Phase B langsam in Phase A einrühren.

Standard O/W-Creme

| Öl-Phase | [%] |
|---|---|
| Paraffinöl | 7,50 |
| Ceteareth-6 und Stearyl-Alkohol | 3,50 |
| Ceteareth-25 | 3,50 |
| Cetylalkohol | 3,50 |
| Glycerinmonostearat s.e. | 2,50 |
| Cetearyloctanoat | 3,20 |
| Methyl- und Propyl-4-hydroxybenzoat | (7 : 3) |
| Tocopheryl Acetat | 1,00 |

| Wasser-Phase | [%] |
|---|---|
| Polymer 1-22 und 22a-e | 1,50 |
| Wasser | 74,60 |
| Imidazolidinyl-Urea | 0,10 |

Herstellung: Einwiegen und unter Rühren der Öl-Phasen und Wasser-Phase getrennt bei einer Temperatur von ca.

80°C homogenisieren. Wasser-Phase langsam in Öl-Phase einrühren. Unter Rühren langsam auf RT abkühlen.

## Patentansprüche

**1.** Polyharnstoff, aufgebaut aus:

a) einem Diamin, das eine Gruppe $-(-CH_2CH_2O-)_n-(C_3H_6-O-)_m$ enthält, wobei die Reihenfolge der Alkylenoxideinheiten beliebig ist und m und n unabhängig voneinander für eine ganze Zahl zwischen 0 und 50 stehen, und m+n zwischen 5 und 60 liegen und
b) mindestens einem aminohaltigen oder hydroxyhaltigen Polysiloxan und
c) mindestens einem Diisocyanat, und
d) optional einem Di-, Tri- oder Tetraamin oder Polyamin, das mindestens eine ionogene Gruppe enthält, und
e) optional einem oder mehreren Diaminen mit einem Molekulargewicht von 6056 bis 6000 g/mol,

wobei der Polyharnstoff mindestens eine ionogene oder ionische Gruppe enthält.

**2.** Verfahren zur Herstellung von Polyharnstoff nach Anspruch 1, wobei die Polymerisation in Alkohol oder Wasser/Alkohol-Lösung bei einer Temperatur kleiner gleich 50°C durchgeführt wird, bei hydroxyhaltigen Polysiloxanen werden zuerst die Komponenten b) und c) miteinander umgesetzt.

**3.** Verwendung von neutralisiertem Polyharnstoff nach Anspruch 1 als filmbildendes Hilfsmittel oder als Additiv für kosmetische Präparate.

**4.** Verwendung von Polyharnstoff nach Anspruch 1 mit ionischen oderfreien ionogenen Gruppen als Neutralisationspolymermittel für Polymere, die ionische oder ionogene Gruppen enthalten.

**5.** Verwendung eines Polyharnstoffs nach einem der vorhergehenden Ansprüche, **dadurch gekennzeichnet, daß** das neutralisierte Produkt eine Glasübergangstemperatur von > 10°C aufweist, als Festigerpolymer oder als Konditionspolymer in der Haarkosmetik, insbesondere für Haarsprays, Haarschäume und Shampoos.

**6.** Kosmetisches und/oder pharmazeutisches Mittel, enthaltend mindestens einen Polyharnstoff nach einem der vorhergehenden Ansprüche.

**7.** Haarbehandlungsmittel, enthaltend

- 0,5-20 Gew.-% eines Polyharnstoffs nach einem der vorhergehenden Ansprüche,
- 50-99,5 Gew.-% eines Lösungsmittels
- 0-70 Gew.-% eines Treibmittels
- 0-10 Gew.-% eines handelsüblichen Haarpolymers,
- 0-0,5 Gew.% einer wasserlöslichen oder dispergierbaren Silikonverbindung,
- 0-40 Gew.-% eines Tensids,
- 0-5,0 Gew.-% eines Emulgators,
- 0-3,0 Gew.-% eines Salzes,
- 0-3,0 Gew.-% eines Verdickers.

**8.** Verwendung eines Polyharnstoffs nach einem der vorhergehenden Ansprüche in kosmetischen und/oder pharmazeutischen Mitteln.

**9.** Verwendung eines Polyharnstoffs nach einem der vorhergehenden Ansprüche in der Textil-, Druck-, Papier-, Leder-, Klebstoff- und Agrarindustrie.

**10.** Verwendung nach Anspruch 8 und/oder 9 als Bestandteil, als Wirkpolymer, Filmbildner, Beschichtungsmittel, Bindemittel, (Co-)Emulgator, Penetrationsinhibitor, Kristallisationsinhibitor und/oder feuchtigkeitshaltende Additive.

**11.** Verwendung nach Anspruch 8 und/oder 9 als viskositätseinstellendes Mittel.